# EUROPÄISCHE PATENTSCHRIFT

(11) **EP 2 758 372 B1**
(45) Veröffentlichungstag und Bekanntmachung des Hinweises auf die Patenterteilung: **17.05.2017**
(21) Anmeldenummer: 12751270.5
(22) Anmeldetag: 23.08.2012
(51) Int. Cl.: C07D 209/86, H01L 51/50, C07D 209/88, C07D 209/96, C07D 405/04, C07D 405/14, C07D 409/04, C07D 409/14, C07D 487/04, C07D 487/10, C07D 491/04, C07D 491/14, C07D 495/04, C07D 495/14, C07F 7/10

(54) **CARBAZOLDERIVATE FÜR ORGANISCHE ELEKTROLUMINESZENZVORRICHTUNGEN**
CARBAZOLE DERIVATIVES FOR ORGANIC ELECTROLUMINESCENT DEVICES
DÉRIVÉS DE CARBAZOLE POUR DES DISPOSITIFS ÉLECTROLUMINESCENTS ORGANIQUES

(30) Priorität: 21.09.2011 EP 11007693
(43) Veröffentlichungstag der Anmeldung: 30.07.2014
(73) Patentinhaber: Merck Patent GmbH, 64293 Darmstadt (DE)
(72) Erfinder: JATSCH, Anja, 60486 Frankfurt am Main (DE); PARHAM, Amir Hossain, 65929 Frankfurt am Main (DE); PFLUMM, Christof, 64291 Darmstadt (DE); STOESSEL, Philipp, 60487 Frankfurt am Main (DE); KROESSER, Jonas Valentin, 60311 Frankfurt am Main (DE); ANEMIAN, Rémi Manouk, Seoul 657-169 (KR); EBERLE, Thomas, 76829 Landau (DE)
(86) Internationale Anmeldenummer: PCT/EP2012/003563
(87) Internationale Veröffentlichungsnummer: WO 2013/041176

(56) Entgegenhaltungen:
- WO-A1-2010/136109
- WO-A1-2012/026780
- WO-A2-2011/057706
- DATABASE CA [Online] CHEMICAL ABSTRACTS SERVICE, COLUMBUS, OHIO, US; 2011, BAE, JAE SUN ET AL: "Preparation of aromatic heteropolycyclic compounds for organic electronic device", XP002684355, gefunden im STN Database accession no. 2011:43266 & KR 2011 002 156 A (LG CHEM, LTD., S. KOREA) 7. Januar 2011 (2011-01-07)
- DATABASE CA [Online] CHEMICAL ABSTRACTS SERVICE, COLUMBUS, OHIO, US; 2012, WANG, GUOFANG ET AL: "Fluorene-fused indoles, emitter layer materials containing them for organic electroluminescent devices, and displays and illumination apparatus using", XP002684349, gefunden im STN Database accession no. 2012:956428 -& JP 2012 126673 A (JNC CORP., JAPAN) 5. Juli 2012 (2012-07-05)
- DATABASE CA [Online] CHEMICAL ABSTRACTS SERVICE, COLUMBUS, OHIO, US; 2012, KIM, BOK YEONG ET AL: "Substituted carbazole-based organic light compound as an electroluminescent material or/and electron/hole transport material for organic light device", XP002684350, gefunden im STN Database accession no. 2012:893981 -& KR 2012 065 214 A (CS ELSOLAR CO., LTD., S. KOREA) 20. Juni 2012 (2012-06-20)
- DATABASE CA [Online] CHEMICAL ABSTRACTS SERVICE, COLUMBUS, OHIO, US; 2012, SHIN, HYO NIM ET AL: "Organic electroluminescent compound as also an hole injection/transport material for organic electroluminescent device", XP002684351, gefunden im STN Database accession no. 2012:537901 -& KR 2012 034 140 A (ROHM AND HAAS ELECTRONIC MATERIALS KOREA LTD., S. KOREA) 10. April 2012 (2012-04-10)
- DATABASE CA [Online] CHEMICAL ABSTRACTS SERVICE, COLUMBUS, OHIO, US; 2012, KIM, YEONG GIL ET AL: "Aromatic hole injecting or transporting material for organic electroluminescent device", XP002684352, gefunden im STN Database accession no. 2012:176927 -& KR 2012 009 984 A (ROHM AND HAAS ELECTRONIC MATERIALS KOREA LTD., S. KOREA) 2. Februar 2012 (2012-02-02)
- DATABASE CA [Online] CHEMICAL ABSTRACTS SERVICE, COLUMBUS, OHIO, US; 2012, YOKOYAMA, NORIMASA ET AL: "Compound having indenocarbazole ring structure, and organic electroluminescent element containing same", XP002684353, gefunden im STN Database accession no. 2012:157698 & WO 2012/014500 A1 (HODOGAYA CHEMICAL CO., LTD., JAPAN) 2. Februar 2012 (2012-02-02)

## Beschreibung

Die vorliegende Erfindung beschreibt Carbazol-Derivate, insbesondere zur Verwendung als Triplettmatrixmaterialien in organischen Elektrolumineszenzvorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden häufig metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer.

Die Eigenschaften von phosphoreszierenden OLEDs werden nicht nur von den eingesetzten Triplettemittern bestimmt. Hier sind insbesondere auch die anderen verwendeten Materialien wie zum Beispiel Matrixmaterialien von besonderer Bedeutung. Verbesserungen dieser Materialien können somit auch zu deutlichen Verbesserungen der OLED-Eigenschaften führen.

Gemäß dem Stand der Technik werden unter anderem Indolocarbazolderivate (z. B. gemäß WO 2007/063754 oder WO 2008/056746) oder Indenocarbazolderivate (z. B. gemäß WO 2010/136109 oder WO 2011/000455), insbesondere solche, die mit elektronenarmen Heteroaromaten wie Triazin substituiert sind, als Matrixmaterialien für phosphoreszierende Emitter verwendet. Weiterhin werden beispielsweise Bisdibenzofuranderivate (z. B. gemäß EP 2301926) als Matrixmaterialien für phosphoreszierende Emitter verwendet. Allerdings besteht bei Verwendung dieser Matrixmaterialien noch Verbesserungsbedarf, insbesondere in Bezug auf die Effizienz, die Lebensdauer und die Betriebsspannung der Vorrichtung.

Aufgabe der vorliegenden Erfindung ist die Bereitstellung von Verbindungen, welche sich für den Einsatz in einer fluoreszierenden oder insbesondere in einer phosphoreszierenden OLED eignen, insbesondere als Matrixmaterial. Insbesondere ist es die Aufgabe der vorliegenden Erfindung, Matrixmaterialien bereitzustellen, welche sich auch für grün und gegebenenfalls auch für blau phosphoreszierende OLEDs eignen, und die zu guter Effizienz, hoher Lebensdauer und geringer Betriebsspannung führen. Gerade die Eigenschaften der Matrixmaterialien haben einen wesentlichen Einfluss auf die Lebensdauer und die Effizienz der organischen Elektrolumineszenzvorrichtung.

Es wurde überraschend gefunden, dass Elektrolumineszenzvorrichtungen, die Verbindungen gemäß der folgenden Formel (1) enthalten, Verbesserungen gegenüber dem Stand der Technik aufweisen, insbesondere beim Einsatz als Matrixmaterialien für phosphoreszierende Dotanden.

Gegenstand der vorliegenden Erfindung ist daher eine Verbindung der folgenden Formel (1), 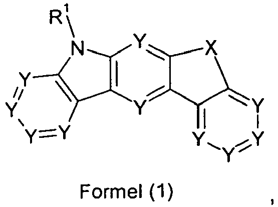 wobei für die verwendeten Symbole gilt:
Y ist bei jedem Auftreten gleich oder verschieden CR oder N;
X ist ausgewählt aus C(R¹)₂, O, S, PR¹, P(=O)R¹ oder BR¹;
R, R¹ ist bei jedem Auftreten gleich oder verschieden H, D, F, Cl, Br, I, N(R²)₂, N(Ar)₂, C(=O)Ar, P(=O)Ar₂, S(=O)Ar, S(=O)₂Ar, CR²=CR²Ar, CN, NO₂, Si(R²)₃, B(OR²)₂, OSO₂R², eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C=C , Si(R²)₂, C=O, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Aralkyl- oder Heteroaralkylgruppe mit 5 bis 40 aromatischen Ringatomen, die durch einen oder mehrere Reste R² substituiert sein kann, oder eine Kombination dieser Systeme; dabei können zwei oder mehrere Substituenten R zusammen mit den Atomen, an die sie gebunden sind, bzw. zwei Substituenten R¹ zusammen mit dem Atom, an das sie gebunden sind, auch miteinander ein mono- oder polycyclisches, aliphatisches oder aromatisches Ringsystem bilden;
   Ar ist bei jedem Auftreten gleich oder verschieden ein aromatisches oder heteroaromatisches Ringsystem, bevorzugt eine Aryl- oder Heteroarylgruppe mit 5 bis 40 aromatischen Ringatomen, das mit einem oder mehreren Resten R³ substituiert sein kann;
   R² ist bei jedem Auftreten gleich oder verschieden H, D, F, Cl, Br, I, N(R³)₂, N(Ar)₂, C(=O)Ar, P(=O)Ar₂, S(=O)Ar, S(=O)₂Ar, CR³=CR³Ar, CN, NO₂, Si(R³)₃, B(OR³)₂, OSO₂R³, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R³ substituiert sein kann, wobei eine oder mehrere nicht benachbarte CH₂-Gruppen durch R³C=CR³, C≡C, Si(R³)₂, C=O, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Aryloxy- oder Heteroaryloxygruppe mit 5 bis 40 aromatischen Ringatomen, die durch einen oder mehrere Reste R³ substituiert sein kann, oder eine Aralkyl- oder Heteroaralkylgruppe mit 5 bis 40 aromatischen Ringatomen, die durch einen oder mehrere Reste R³ substituiert sein kann, oder eine Kombination dieser Systeme;
   R³ ist bei jedem Auftreten gleich oder verschieden H, D oder ein aliphatischer Kohlenwasserstoffrest mit 1 bis 20 C-Atomen oder eine Aryl- oder Heteroarylgruppe mit 5 bis 40 Ringatomen oder eine Kombination dieser Gruppen;
mit der Maßgabe, dass, wenn eine oder mehrere der Gruppen R, R¹, R², R³, Ar oder Ar¹ Heteroarylgruppen enthalten, welche nicht den Formeln (2) oder (3) entsprechen, es sich dabei nicht um elektronenarme Heteroarylgruppen handelt, wobei eine elektronenarme Heteroarylgruppe eine 5-Ring-Heteroarylgruppe mit mindestens zwei Heteroatomen oder eine 6-Ring-Heteroarylgruppe mit mindestens einem Heteroatom ist und an diese Gruppen noch weitere 6-Ring-Aryl- oder 6-Ring-Heteroarylgruppen ankondensiert sein können;
dadurch gekennzeichnet, dass mindestens eine Gruppe R vorhanden ist, die gleich oder verschieden bei jedem Auftreten für eine Gruppe der folgenden Formel (2) steht, 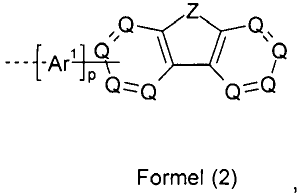 wobei die gestrichelte Bindung die Verknüpfung der Gruppe der Formel (2) andeutet, R² die oben genannten Bedeutungen aufweist und weiterhin gilt:
- Q: ist C, wenn die Gruppe der Formel (2) über diese Gruppe mit Ar¹ bzw. mit dem restlichen Molekül verknüpft ist; bzw. ist gleich oder verschieden bei jedem Auftreten CR² oder N in den sonstigen Fällen;
- Z: ist NR²;
- Ar¹: ist ein bivalentes aromatisches oder heteroaromatisches Ring-system mit 5 bis 40 aromatischen Ringatomen, welches durch einen oder mehrere Reste R² substituiert sein kann;
- p: ist 0 oder 1;
und/oder dass mindestens eine Gruppe R¹ vorhanden ist, die für eine Gruppe der folgenden Formel (3) steht, 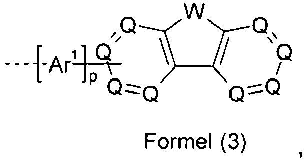 wobei die gestrichelte Bindung die Verknüpfung der Gruppe der Formel (3) andeutet, R², Ar¹, Q und p die oben genannten Bedeutungen aufweisen und weiterhin gilt:
- W: ist NR², O oder S;
dabei sind Verbindungen der folgenden allgemeinen Formel von der Erfindung ausgenommen: 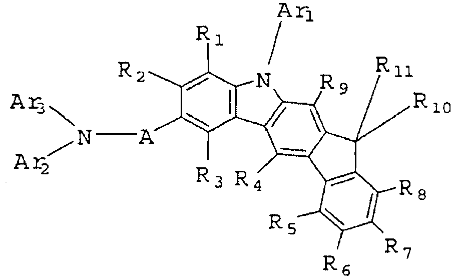 wobei für die verwendeten Symbole gilt:
- A: ist eine divalente substituierte oder unsubstituierte aromatische Kohlenwasserstoffgruppe, ein divalenter substituierter oder unsubstituierter aromatischer heterocyclischer Ring oder eine divalente substituierte oder unsubstituierte kondensierte polycyclische aromatische Gruppe;
- Ar₁, Ar₂, Ar₃: sind gleich oder verschieden und stehen für eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe, wobei A und Ar₂ über eine Einfachbindung aneinander gebunden sind und so einen Ring bilden;
- R₁ bis R₉: sind gleich oder verschieden und stehen für ein Wasserstoffatom, ein Deuteriumatom, ein Fluoratom, ein Chloratom, Cyano, Nitro, lineares oder verzweigtes Alkyl mit 1 bis 6 Kohlenstoffatomen, das einen Substituenten tragen kann, Cycloalkyl mit 5 bis 10 Kohlenstoffatomen, das einen Substituierten tragen kann, lineares oder verzweigtes Alkenyl mit 2 bis 6 Kohlenstoffatomen, das einen Substituenten tragen kann, lineares oder verzweigtes Alkyloxy mit 1 bis 6 Kohlenstoffatomen, das einen Substituenten tragen kann, Cycloalkyloxy mit 5 bis 10 Kohlenstoffatomen, das einen Substituenten tragen kann, eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder substituiertes oder unsubstituiertes Aryloxy, welche über eine Einfachbindung, substituiertes oder unsubstituiertes Methylen, ein Sauerstoffatom oder ein Schwefelatom aneinander binden und so einen Ring bilden können; und
- R₁₀ und R₁₁: sind gleich oder verschieden und stehen für lineares oder verzweigtes Alkyl mit 1 bis 6 Kohlenstoffatomen, das einen Substituenten tragen kann, Cycloalkyl mit 5 bis 10 Kohlenstoffatomen, das einen Substituenten tragen kann, lineares oder verzweigtes Alkenyl mit 2 bis 6 Kohlenstoffatomen, das einen Substituenten tragen kann, lineares oder verzweigtes Alkyloxy mit 1 bis 6 Kohlenstoffatomen, das einen Substituenten tragen kann, Cycloalkyloxy mit 5 bis 10 Kohlenstoffatomen, das einen Substituenten tragen kann, eine substituierte oder unsubstituierte aromatische Kohlenwasserstoffgruppe, eine substituierte oder unsubstituierte aromatische heterocyclisch Gruppe, eine substituierte oder unsubstituierte kondensierte polycyclische aromatische Gruppe oder substituiertes oder unsubstituiertes Aryloxy, welche über eine Einfachbindung, substituiertes oder unsubstituiertes Methylen, ein Sauerstoffatom oder ein Schwefelatom aneinander binden können und so einen Ring bilden.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Thiophen, etc., oder eine kondensierte (anellierte) Aryl- oder Heteroarylgruppe, beispielsweise Naphthalin, Anthracen, Phenanthren, Dibenzofuran, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 80 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Weiterhin werden miteinander durch Einfachbindung verknüpfte Aromaten, wie zum Beispiel Biphenyl, als aromatisches Ringsystem im Sinne dieser Anmeldung bezeichnet.

Eine elektronenarme Heteroarylgruppe im Sinne der vorliegenden Erfindung ist definiert als 5-Ring-Heteroarylgruppe mit mindestens zwei Heteroatomen, beispielsweise Imidazol, Oxazol, Oxadiazol, etc., oder als 6-Ring-Heteroarylgruppe mit mindestens einem Heteroatom, beispielsweise Pyridin, Pyrimidin, Pyrazin, Triazin, etc.. Dabei können an diese Gruppen auch noch weitere 6-Ring-Aryl- oder 6-Ring-Heteroarylgruppen ankondensiert sein, wie beispielsweise in Benzimidazol oder Chinolin.

Im Rahmen der vorliegenden Erfindung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkenyl-, Alkinyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 80 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme. Dabei können diese Gruppen jeweils durch die oben genannten Reste substituiert sein.

In einer bevorzugten Ausführungsform der Erfindung steht der Rest R¹, der an das Stickstoffatom bindet, für ein aromatisches oder heteroaromatisches Ringsystem mit 5 bis 60 aromatischen Ringatomen, bevorzugt mit 6 bis 24 aromatischen Ringatomen, das auch durch einen oder mehrere Reste R² substituiert sein kann, oder für eine Gruppe der oben genannten Formel (3).

In einer weiteren bevorzugten Ausführungsform der erfindungsgemäßen Verbindungen steht X für C(R¹)₂. In diesem Fall stehen die Reste R¹, die an dieses Kohlenstoffatom binden, gleich oder verschieden bei jedem Auftreten bevorzugt für eine geradkettige Alkylgruppe mit 1 bis 10 C-Atomen oder eine verzweigte oder cyclische Alkylgruppe mit 3 bis 10 C-Atomen, die jeweils mit einem oder mehreren Resten R² substituiert sein kann, wobei eine oder mehrere nicht benachbarte CH₂-Gruppen durch R²C=CR², C=C , Si(R²)₂, C=O, O, S oder CONR² ersetzt sein können und wobei ein oder mehrere H-Atome durch D, F oder CN ersetzt sein können, oder für ein aromatisches oder heteroaromatisches Ringsystem mit 5 bis 60, bevorzugt mit 5 bis 24 aromatischen Ringatomen, das auch durch einen oder mehrere Reste R² substituiert sein kann. Dabei können die beiden Reste R¹, die an dasselbe Kohlenstoffatom binden, auch miteinander ein aliphatisches oder aromatisches Ringsystem bilden.

In einer weiteren bevorzugten Ausführungsform der Erfindung steht maximal eine Gruppe Y pro Cyclus für N und die verbleibenden Gruppen Y stehen gleich oder verschieden bei jedem Auftreten für CR. Besonders bevorzugt steht Y gleich oder verschieden bei jedem Auftreten für CR.

Bevorzugte Ausführungsformen der Verbindungen gemäß Formel (1) sind daher die Verbindungen gemäß der folgenden Formel (5), 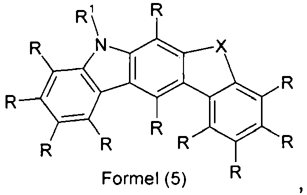 wobei die verwendeten Symbole die oben genannten Bedeutungen aufweisen, und, wie oben beschrieben, mindestens eine der Gruppen der Formel (2) oder (3) vorhanden ist.

Besonders bevorzugte Ausführungsformen der Strukturen gemäß Formel (5) sind die Strukturen gemäß der folgenden Formel (5a), 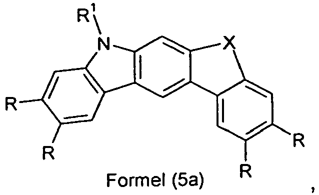 wobei die verwendeten Symbole die oben genannten Bedeutungen aufweisen.

Besonders bevorzugt sind Strukturen der folgenden Formeln (6), (7) und (8), 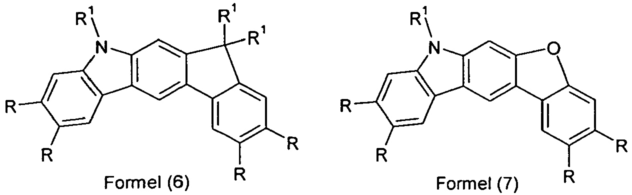 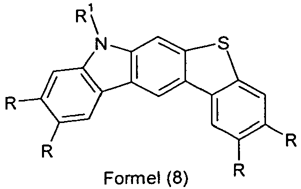 und ganz besonders bevorzugt sind die Verbindungen der folgenden Formeln (6a), (7a) und (8a), 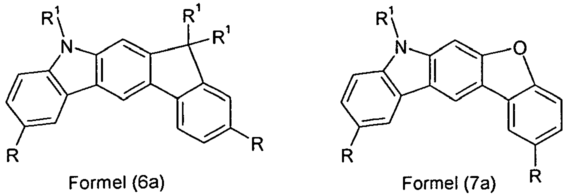 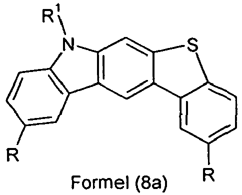 wobei die verwendeten Symbole die oben genannten Bedeutungen aufweisen.

Insbesondere bevorzugt sind die Verbindungen der folgenden Formeln (6b), (7b) und (8b), 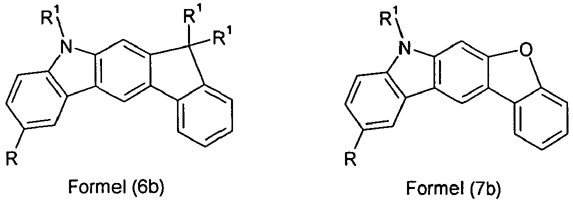 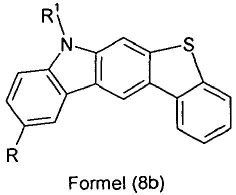 wobei die verwendeten Symbole die oben genannten Bedeutungen aufweisen.

Dabei können auch zwei Reste R¹, die in Formel (6) bzw. (6a) bzw. (6b) an dasselbe C-Atom gebunden sind, zusammen mit dem Kohlenstoffatom, an das sie gebunden sind, ein aliphatisches, aromatisches oder heteroaromatisches Ringsystem bilden, beispielsweise ein Fluoren, und so insgesamt ein Spirosystem aufspannen.

Besonders bevorzugt sind Indenocarbazolderivate, also die Verbindungen der Formel (6) bzw. (6a) bzw. (6b).

Wie oben beschrieben, enthält die erfindungsgemäße Verbindung mindestens eine Gruppe R gemäß Formel (2) und/oder mindestens eine Gruppe R¹ gemäß Formel (3).

In einer weiteren bevorzugten Ausführungsform der Erfindung enthält die Verbindung gemäß Formel (1) eine, zwei oder drei Gruppen gemäß einer oder mehreren der Formeln (2) bis (3), besonders bevorzugt eine oder zwei Gruppen gemäß einer oder mehreren der Formeln (2) bis (3), ganz besonders bevorzugt genau eine Gruppe gemäß einer der Formeln (2) bis (3).

Wenn die erfindungsgemäße Verbindung eine Gruppe der Formel (3) aufweist, so ist diese Gruppe der Formel (3) bevorzugt an das Stickstoffatom der Verbindung gebunden, also bevorzugt nicht an die Gruppe X.

Im Folgenden werden die bevorzugten Ausführungsformen der Gruppen gemäß den Formeln (2) bis (3) beschrieben.

In einer bevorzugten Ausführungsform der Erfindung steht in den Gruppen der Formeln (2) bis (3) jeweils maximal eine Gruppe Q pro Cyclus für N und die verbleibenden Gruppen Q stehen gleich oder verschieden bei jedem Auftreten für CR² bzw. für C, wenn an diese Gruppe die Gruppe Ar¹ bzw. das restliche Molekül angeknüpft ist. In einer besonders bevorzugten Ausführungsform der Erfindung steht Q für C, wenn die Gruppe der Formel (2) oder (3) über diese Gruppe mit Ar¹ bzw. mit dem restlichen Molekül verknüpft ist, und die verbleibenden Gruppen Q stehen gleich oder verschieden für CR².

Bevorzugte Ausführungsformen der Formeln (2) bis (3) sind daher die Gruppen der folgenden Formeln (2a) bis (3a), 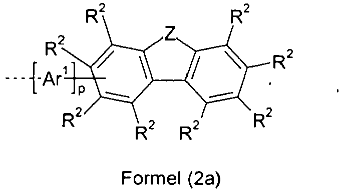 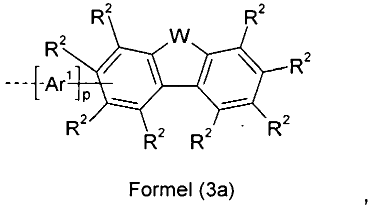 wobei die gestrichelte Bindung die Verknüpfung der Gruppe mit dem restlichen Molekül andeutet und die verwendeten Symbole und Indizes die oben genannten Bedeutungen aufweisen und in Formel (2a) und (3a) an der Position, an die die Gruppe Ar¹ bzw. das restliche Molekül verknüpft ist, keine Gruppe R² gebunden ist.

Besonders bevorzugte Ausführungsformen der Formeln (2a) bis (3a) sind die Strukturen der Formeln (2b), (2c), (2d), (3b), (3c) und (3d), 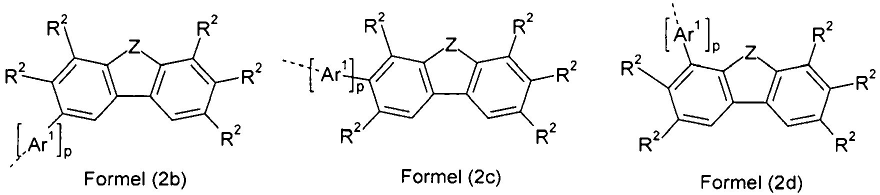 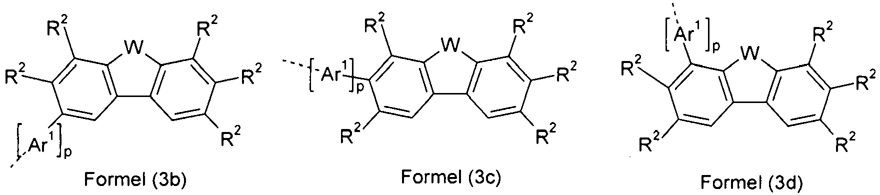 wobei die gestrichelte Bindung die Verknüpfung der Gruppe mit dem restlichen Molekül andeutet und die weiteren verwendeten Symbole und Indizes die oben genannten Bedeutungen aufweisen.

Bevorzugt steht in den oben genannten Strukturen Z und W für NR², wobei R² für ein aromatisches oder heteroaromatisches Ringsystem steht gemäß der oben genannten Definition, welches auch durch die oben genannten Reste substituiert sein kann.

Bevorzugt stehen weiterhin in den oben genannten Strukturen die Reste R², welche an ein Kohlenstoffatom gebunden sind, für H.

In einer weiteren bevorzugten Ausführungsform der Erfindung ist der Index p = 0.

Besonders bevorzugt sind die Strukturen der Formeln (2b) und (3b).

Wenn eine Gruppe Ar¹ vorhanden ist, steht diese bevorzugt für ein bivalentes aromatisches oder heteroaromatisches Ringsystem mit 6 bis 24 aromatischen Ringatomen, welches bevorzugt keine kondensierte Aryl- oder Heteroarylgruppe mit mehr als zwei direkt aneinander kondensierten Sechsringen enthält. Bevorzugte Gruppen Ar¹ sind ausgewählt aus der Gruppe bestehend aus ortho-, meta- oder para-Benzol, ortho-, meta- oder para-Biphenyl, Terphenyl, insbesondere ortho-, meta- oder para-Terphenyl, Quaterphenyl, insbesondere ortho-, meta- oder para-Quaterphenyl, Fluoren, Furan, Benzofuran, Dibenzofuran, Dibenzothiophen, Pyrrol, Indol oder Carbazol. Dabei können diese Gruppen durch einen oder mehrere Reste R² substituiert sein, sind aber bevorzugt unsubstituiert. Wenn Ar¹ für Fluoren steht, so ist dieses bevorzugt in 9-Position durch zwei Alkylgruppen mit jeweils 1 bis 10 C-Atomen substituiert.

Die oben genannten Ausführungsformen der Erfindung sind beliebig miteinander kombinierbar. Insbesondere sind die oben aufgeführten allgemeinen Formeln (1) bzw. die bevorzugten Ausführungsformen beliebig mit den Formeln (2) bis (3) bzw. den entsprechenden bevorzugten Ausführungsformen sowie mit den oben genannten bevorzugten Ausführungsformen für die weiteren Symbole und Indizes beliebig kombinierbar. In einer bevorzugten Ausführungsform der Erfindung treten die oben genannten Bevorzugungen gleichzeitig auf. So ist es insbesondere möglich, jede der Formeln (5), (5a), (6), (6a), (6b), (7), (7a), (7b), (8), (8a) bzw. (8b) mit jeder der Formeln (2a), (2b), (2c), (2d), (3a), (3b), (3c) bzw. (3d) zu kombinieren.

Wenn in der Verbindung der allgemeinen Formel (1) ein oder mehrere Reste R vorhanden sind, die ungleich H oder D sind und die nicht für eine Gruppe der Formel (2) oder (3) stehen, so sind diese Reste bevorzugt ausgewählt aus der Gruppe bestehend aus N(Ar)₂, bevorzugt Diphenylamino, einem substituierten oder unsubstituierten Arylamin, einer geradkettigen Alkylgruppe mit 1 bis 20 C-Atomen, bevorzugt 1 bis 10 C-Atomen, einer verzweigten Alkylgruppe mit 3 bis 20 C-Atomen, bevorzugt 1 bis 10 C-Atomen, oder einem aromatischen oder heteroaromatischen Ringsystem mit 5 bis 40 aromatischen Ringatomen, das durch ein oder mehrere Reste R² substituiert sein kann. Dabei ist das aromatische oder heteroaromatische Ringsystem vorzugsweise ausgewählt aus substituiertem oder unsubstituiertem Phenyl, Naphthyl, Thiophen, Dibenzothiophen, Dibenzofuran Triphenylamin oder Kombinationen dieser Gruppen, welche jeweils mit einem oder mehreren Resten R² substituiert sein können.

In einer weiteren bevorzugten Ausführungsform der Erfindung steht X in Formel (1) für C(R¹)₂, wobei die Reste R¹ miteinander ein Ringsystem bilden, so dass eine Struktur der folgenden Formel (9) oder (10) entsteht: 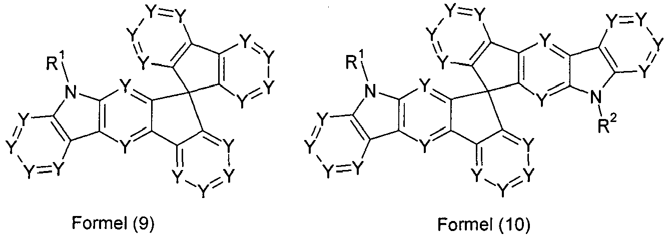 wobei die verwendeten Symbole die oben genannten Bedeutungen aufweisen und Y bevorzugt gleich oder verschieden bei jedem Auftreten für CR steht.

In nochmals einer weiteren bevorzugten Ausführungsform der Erfindung bilden zwei benachbarte Reste R am Grundkörper der Formel (1) ein aromatisches Ringsystem, so dass eine Struktur der folgenden Formel (11) oder (12) entsteht: 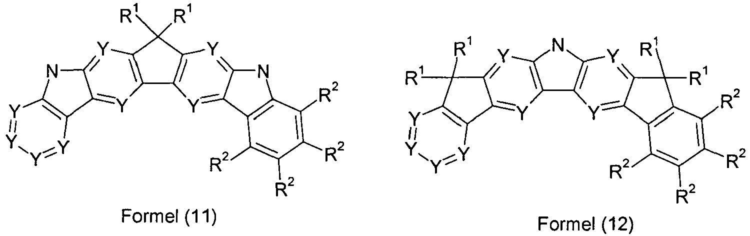 wobei die verwendeten Symbole die oben genannten Bedeutungen aufweisen und Y bevorzugt gleich oder verschieden bei jedem Auftreten für CR steht.

Für die Verbindungen gemäß den Formeln (9) bis (12) gelten dieselben Bevorzugungen, wie oben beschrieben.

Beispiele für erfindungsgemäße Verbindungen sind die nachstehend gezeigten Strukturen.

| | | |
|---|---|---|
| 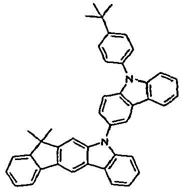 | 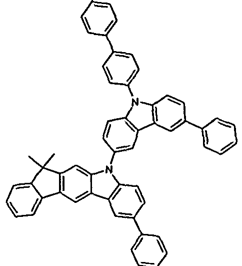 | 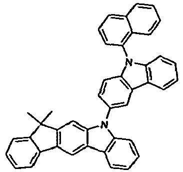 |
| (1) | (2) | (3) |
| 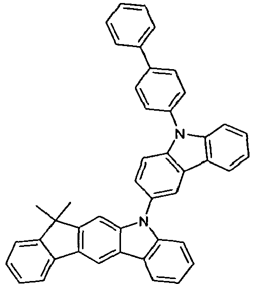 | 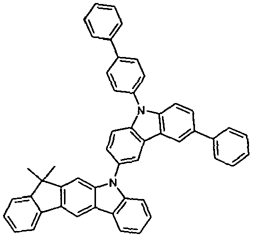 | 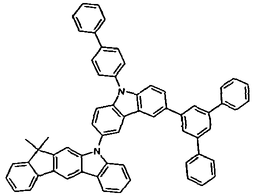 |
| (4) | (5) | (6) |
| 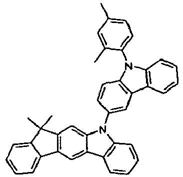 | 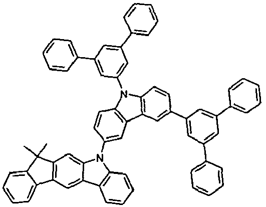 | 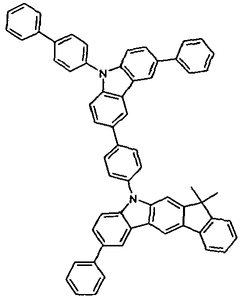 |
| (7) | (8) | (9) |
| 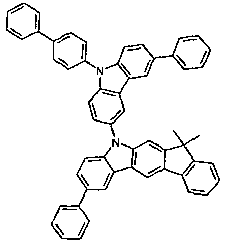 | 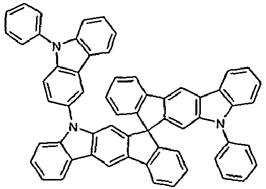 | 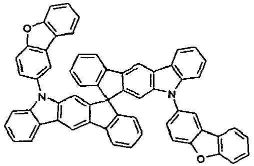 |
| (10) | (11) | (12) |
| 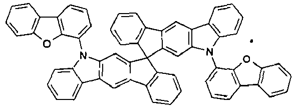 | 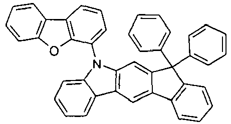 | 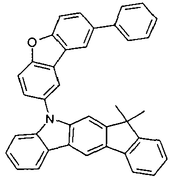 |
| (13) | (14) | (15) |
| 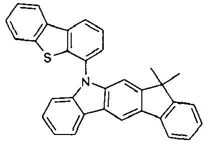 | 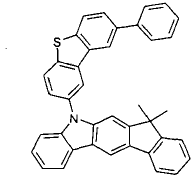 | 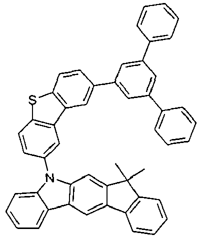 |
| (16) | (17) | (18) |
| 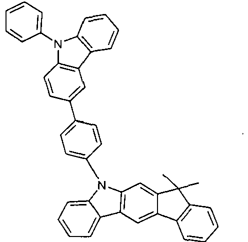 | 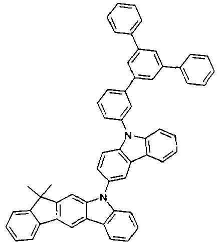 | 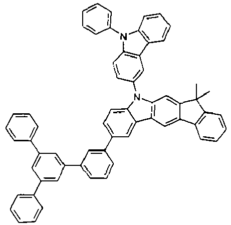 |
| (19) | (20) | (21) |
| 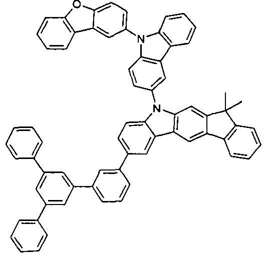 | | 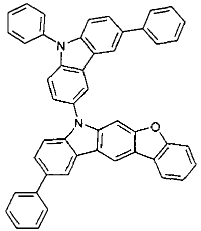 |
| (22) | | (24) |
| 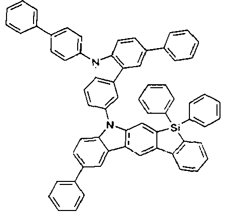 | 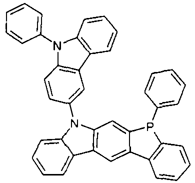 | 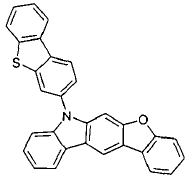 |
| (25) | (26) | (27) |
| 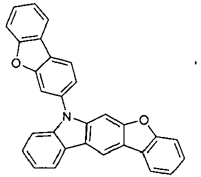 | 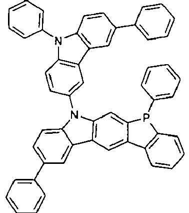 | 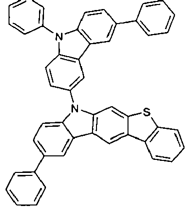 |
| (28) | (29) | (30) |
| 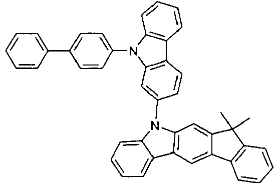 | 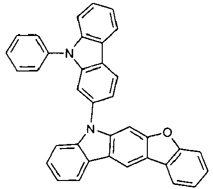 | 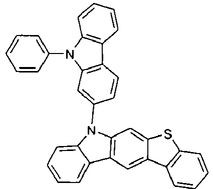 |
| (31) | (32) | (33) |
| | 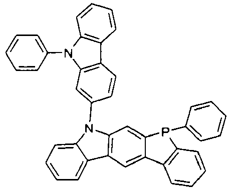 | 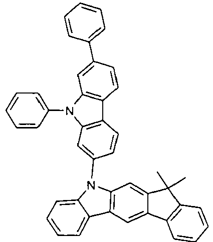 |
| | (35) | (36) |
| 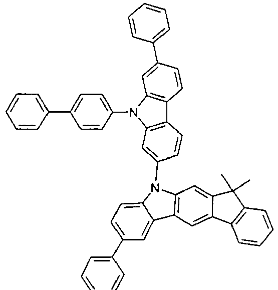 | 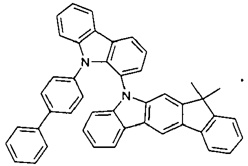 | 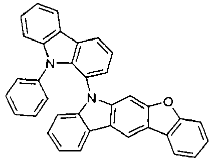 |
| (37) | (38) | (39) |
| 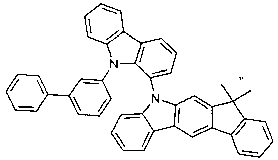 | 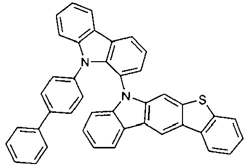 | |
| (40) | (41) | |
| | | 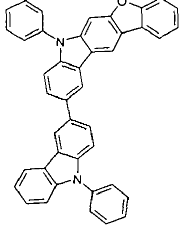 |
| | | (54) |
| | | 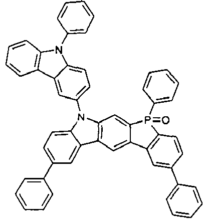 |
| | | (60) |
| | | 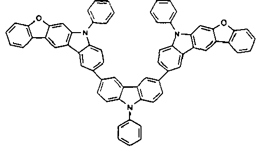 |
| | | (63) |
| 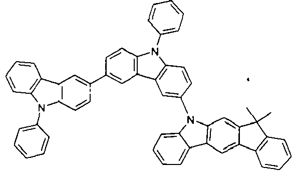 | | |
| (76) | | |
| | | 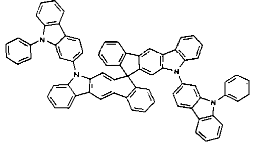 |
| | | (81) |
| | 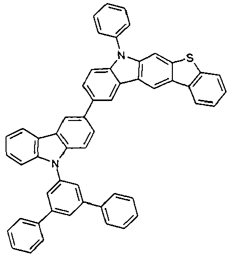 | 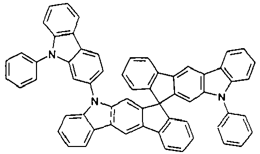 |
| | (83) | (84) |
| | | 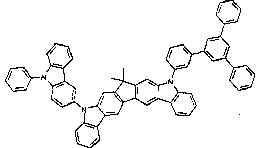 |
| | | (87) |
| 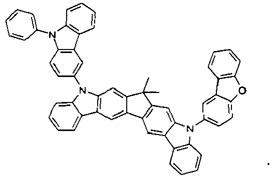 | 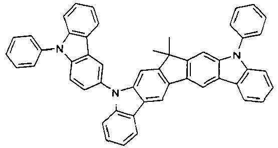 | 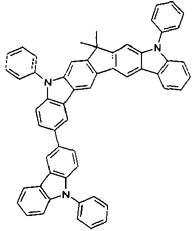 |
| (88) | (89) | (90) |
| | | 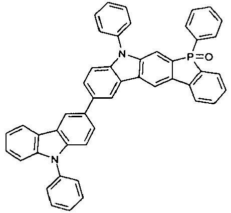 |
| | | (93) |
| | 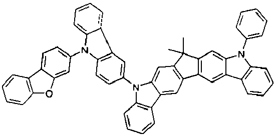 | 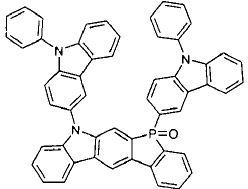 |
| | (95) | (96) |
| 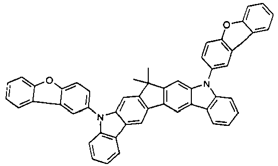 | 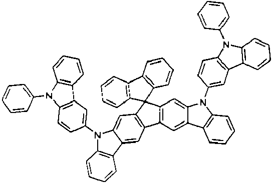 | 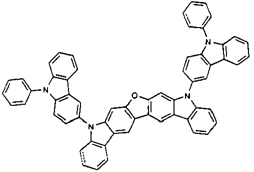 |
| (97) | (98) | (99) |
| 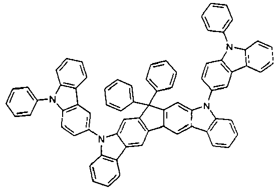 | 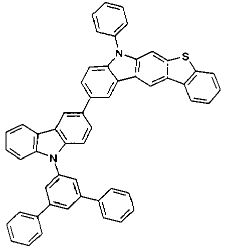 | 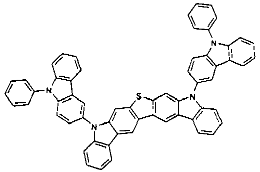 |
| (100) | (101) | (102) |
| 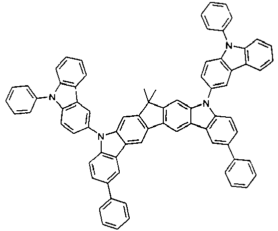 | 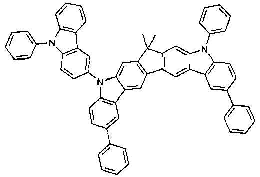 | 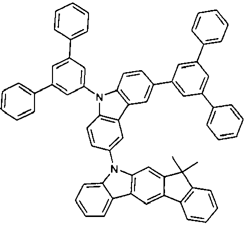 |
| (103) | (104) | (105) |
| 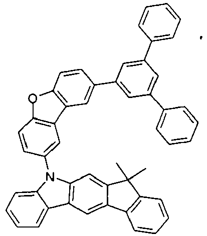 | 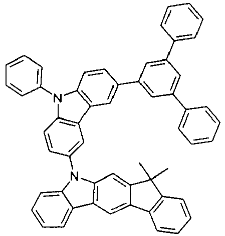 | 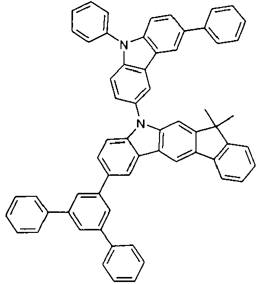 |
| (106) | (107) | (108) |
| 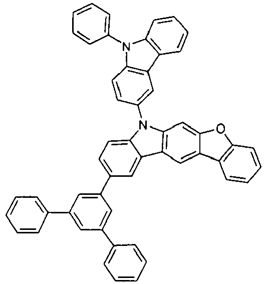 | 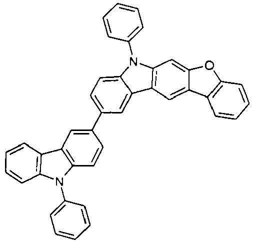 | 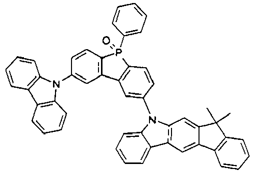 |
| (109) | (110) | (111) |

Die erfindungsgemäßen Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden. Dabei wird bevorzugt eine Einheit gemäß einer der Formeln (2) bis (3) an den Indenocarbazol-Grundkörper bzw. das entsprechende Derivat mit O oder S in der Brücke durch eine Suzuki-Kupplung, eine Ullmann-Kupplung oder eine Hartwig-Buchwald-Kupplung eingeführt.

Ein weiterer Gegenstand der Erfindung ist daher ein Verfahren zur Herstellung einer erfindungsgemäßen Verbindung, dadurch gekennzeichnet, dass die Gruppe der Formel (2) oder (3) durch eine Suzuki-Kupplung, eine Ullmann-Kupplung oder durch eine Hartwig-Buchwald-Kupplung eingeführt wird.

Ein weiterer Gegenstand der vorliegenden Erfindung sind Mischungen enthaltend mindestens eine erfindungsgemäße Verbindung und mindestens eine weitere Verbindung. Die weitere Verbindung kann beispielsweise ein fluoreszierender oder phosphoreszierender Dotand sein, wenn
die erfindungsgemäße Verbindung als Matrixmaterial verwendet wird, insbesondere ein phosphoreszierender Dotand. Geeignete Dotanden sind unten im Zusammenhang mit den organischen Elektrolumineszenzvorrichtungen aufgeführt und sind auch für die erfindungsgemäßen Mischungen bevorzugt.

Für die Verarbeitung aus Lösung bzw. aus flüssiger Phase, beispielsweise durch Spin-Coating oder durch Druckverfahren, sind Lösungen bzw. Formulierungen der erfindungsgemäßen Verbindungen bzw. Mischungen erforderlich. Es kann bevorzugt sein, Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Ein weiterer Gegenstand der vorliegenden Erfindung ist daher eine Formulierung, insbesondere eine Lösung, eine Suspension oder eine Miniemulsion, enthaltend mindestens eine erfindungsgemäße Verbindung oder Mischung und ein oder mehrere Lösemittel, insbesondere organische Lösemittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) oder gemäß den bevorzugten Ausführungsformen als Matrixmaterial für fluoreszierende oder phosphoreszierende Emitter, insbesondere für phosphoreszierende Emitter, und/oder in einer Elektronentransportschicht und/oder in einer elektronenblockierenden bzw. exzitonenblockierenden Schicht und/oder in einer Lochtransportschicht, je nach genauer Substitution. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1) bzw. gemäß den bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) bzw. gemäß den bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1, insbesondere aus einem angeregten Triplettzustand. Im Sinne dieser Anmeldung sollen alle lumineszierenden Übergangsmetallkomplexe und lumineszierenden Lanthanidkomplexe, insbesondere alle Iridium-, Platin- und Kupferkomplexe als phosphoreszierende Verbindungen angesehen werden.

Die Mischung aus der Verbindung gemäß Formel (1) bzw. gemäß den bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. gemäß den bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der Verbindung gemäß Formel (1) bzw. gemäß den bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (1) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder gemäß der nicht offen gelegten Anmeldung EP 11003232.3, Triphenylenderivaten, z. B. gemäß WO 2012/048781, oder Lactame, z. B. gemäß WO 2011/116865 oder WO 2011/137951. Ebenso kann ein weiterer phosphoreszierender Emitter, welcher kürzerwellig als der eigentliche Emitter emittiert, als Co-Host in der Mischung vorhanden sein.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339 oder WO 2012/007086 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Lochtransportschicht oder in einer Lochinjektionsschicht oder in einer Exzitonen- bzw. Elektronenblockierschicht einzusetzen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne efinderisches Zutun alle für organische Elektrolumineszenzvorrichtungen bekannten Materialien in Kombination mit den erfindungsgemäßen Verbindungen gemäß Formel (1) bzw. gemäß den bevorzugten Ausführungsformen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen weisen bei Verwendung in organischen Elektrolumineszenzvorrichtungen einen oder mehrere der folgenden überraschenden Vorteile gegenüber dem Stand der Technik auf:
1. Die Leistungseffizienz entsprechender Vorrichtungen wird höher im Vergleich zu Systemen gemäß dem Stand der Technik.
2. Die Stabilität entsprechender Vorrichtungen wird höher im Vergleich zu Systemen gemäß dem Stand der Technik, was sich vor allem in einer deutlich höheren Lebensdauer zeigt.
3. Die erfindungsgemäßen organische Elektroluminszenzvorrichtungen weisen eine verringerte Betriebsspannung auf.
4. Wenn die erfindungsgemäßen Verbindungen als Matrixmaterial für phosphoreszierende Emitter verwendet werden, ist es möglich, mit nur einer geringen Emitterkonzentration im Bereich von weniger als 10 Vol.% bereits sehr gute Ergebnisse zu erzielen.
5. Die erfindungsgemäßen Verbindungen weisen eine sehr gute thermische Stabilität auf.

Die Erfindung wird nun durch die nachfolgenden Beispiele näher erläutert, ohne sie dadurch einschränken zu wollen.

### Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Zu den literaturbekannten Verbindungen sind jeweils auch die entsprechenden CAS-Nummern angegeben.

### Teil A: Synthese der Vorstufen

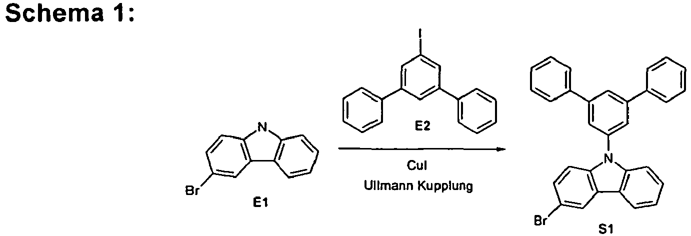

### S1: 3-Brom-9-[1,1';3',1"]terphenyl-5'-yl-9H-carbazol

10 g (41 mmol) 3-Brom-9H-carbazol (CAS 86-74-8) und 16 g (45 mmol, 1.1 eq) 5'-Iod-[1,1';3',1"]terphenyl werden zusammen mit 51 g (270 mmol, 6.6 eq) elementarem Kupfer, 115 g (540 mmol, 13 eq) Kaliumcarbonat und 0.52 g (4.5 mmol, 0.11 eq) 18-Krone-6 in 500 ml p-Xylol gelöst und unter Rückfluss erhitzt. Nach beendeter Reaktion wird das Gemisch dreimal mit Wasser extrahiert, die organische Phase über Natriumsulfat getrocknet, das Lösungsmittel im Vakuum entfernt und der erhaltene Feststoff mittels Säulenchromatogrpahie (Ethylacetat/Heptan) aufgereinigt. Es werden 17 g (36 mmol, 53%) des Produkts erhalten.

### Analog werden folgende Synthons dargestellt:

| **Bsp.** | **E1** | **E2** | **Produkt** | **Ausbeute [%]** |
|---|---|---|---|---|
| **S2** | 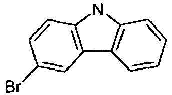 | 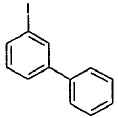 | 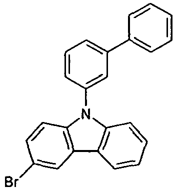 | 48 |
| | CAS 86-74-8 | CAS 20442-79-9 | | |
| **S3** | 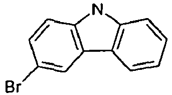 | 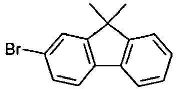 | 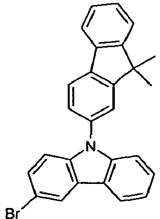 | 63 |
| | CAS 86-74-8 | CAS 28320-31-2 | | |
| **S4** | 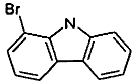 | 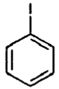 | 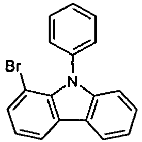 | 54 |
| | CAS 16807-11-7 | CAS 591-50-4 | | |

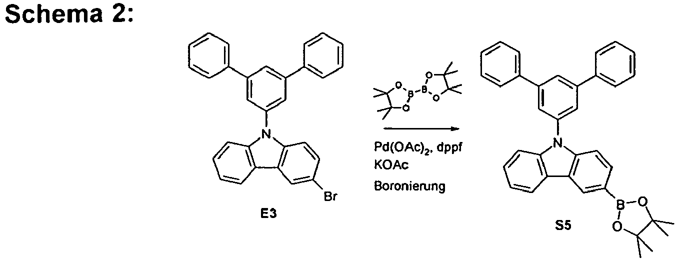

### S5: 9-[1,1';3',1"]-Terphenyl-5'-yl-3-(4,4,5,5-tetramethyl-[1,3,2]dioxaborolan-2-yl)-9H-carbazol

20 g (42 mmol) 3-Brom-9-[1,1';3',1"]terphenyl-5'-yl-9H-carbazol **S1**, 13 g (50 mmol, 1.2 eq.) Bis(pinacolato)-diboran (CAS 73183-34-4) und 12 g (130 mmol, 3 eq.) Kaliumacetat werden in 300 ml 1,4-Dioxan vorgelegt und für 30 Minuten mit Stickstoff entgast. Anschließend werden 470 mg (0.84 mmol, 0.02 eq) 1,1'-Bis(diphenylphosphino)-ferrocen und 190 mg (0.84 mmol, 0.02 eq) Palladium(II)acetat zugegeben und auf 100 °C Innentemperatur erhitzt. Nach beendeter Reaktion wird der Ansatz mit Ethylacetat versetzt und dreimal mit Wasser extrahiert. Die organische Phase wird eingeengt und der Boronester aus Heptan gefällt. Nach Umkristallisation aus Acetonitril werden 20 g (38 mmol, 91%) des Produkts erhalten.

### Analog werden folgende Synthons hergestellt:

| **Bsp**. | **Edukt E3** | **Produkt** | **Ausbeute [%]** |
|---|---|---|---|
| **S6** | 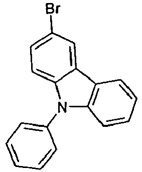 | 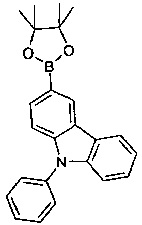 | 97 |
| | CAS 1153-85-1 | | |
| **S7** | 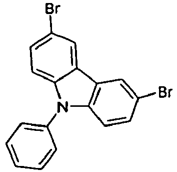 | 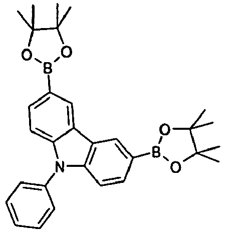 | 89 |
| | CAS 57103-20-5 | 2.4 Äquivalente des Diborans | |

### Teil B: Synthese der erfindungsgemäßen Verbindungen

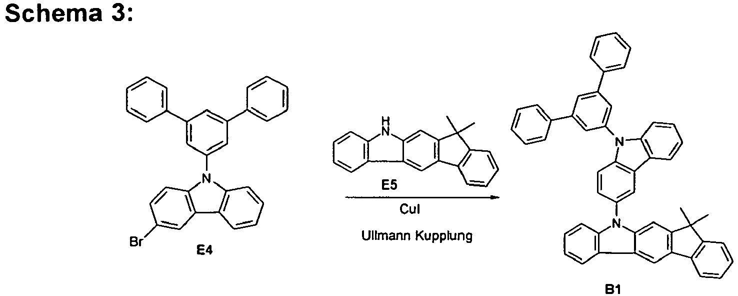

### B1: 12,12-Dimethyl-10-(9-[1,1';3',1"]terphenyl-5'-yl-9H-carbazol-3-yl)-10,12-dihydro-10-aza-indeno[2,1-b]fluoren

7.6 g (33 mmol) 12,12-Dimethyl-10,12-dihydro-10-aza-indeno[2,1-b]-fluoren (WO 2010/136109), 17 g (36 mmol, 1.1 eq) 3-Brom-9-[1,1';3',1"]-terphenyl-5'-yl-9H-carbazol **S1** und 12.1 g (12 mmol, 0.36 eq) Kupfer(I)-iodid werden mit 150 g (706 mmol, 4 eq) Kaliumphosphat in 1 L 1,4-Dioxan suspendiert. Anschließend wird das Reaktionsgemisch für 30 Minuten entgast und unter Schutzgas 17.6 ml (147 mmol, 0.83 eq) *trans-*Cyclohexylamin zugegeben. Der Ansatz wird 12 h unter Rückfluss erhitzt und nach beendeter Reaktion mit Dichlormethan versetzt. Der ausgefallene Feststoff wird abgesaugt, in Toluol gelöst und über Kieselgel filtriert. Nach Entfernen des Lösungsmittels im Vakuum wird der Rückstand mehrmals aus Toluol/Heptan umkristallisiert und abschließend sublimiert. Es werden 17.6 g (33.5 mmol, 57 %) eines farblosen Feststoffs mit einer HPLC-Reinheit >99.9% erhalten.

### Analog B1 werden folgende Verbindungen dargestellt:

| **Bsp.** | **E5** | **E4** | **Produkt** | **Ausbeute [%]** |
|---|---|---|---|---|
| **B2** | 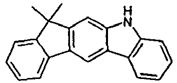 | 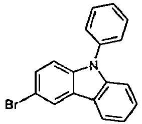 | 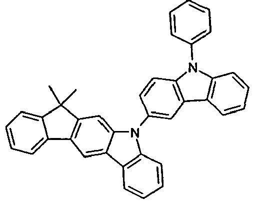 | 54 |
| | WO 2010/136109 | CAS 1153-85-1 | | |
| **B3** | 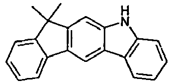 | 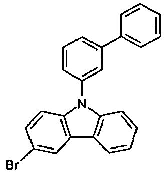 | 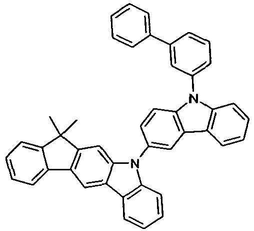 | 74 |
| | WO 2010/136109 | S2 | | |
| **B4** | 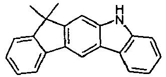 | 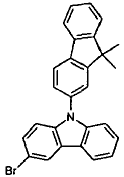 | 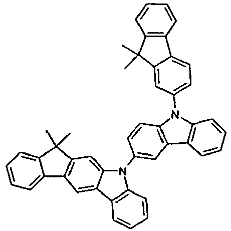 | 65 |
| | WO 2010/136109 | **S3** | | |
| **B5** | 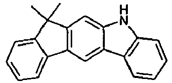 | 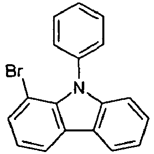 | 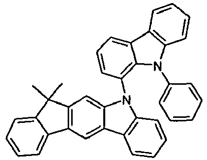 | 53 |
| | WO 2010/136109 | **S4** | | |
| **B6** | 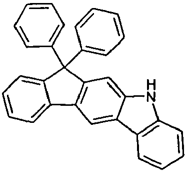 | 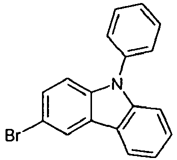 | 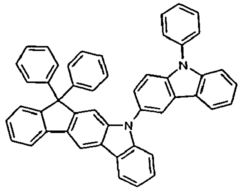 | 59 |
| | WO 2010/136109 | CAS 1153-85-1 | | |
| **B7** | 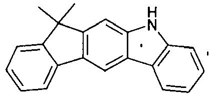 | 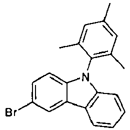 | 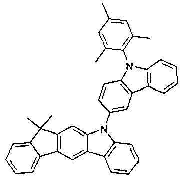 | 49 |
| | WO 2010/136109 | CAS 1141017-78-8 | | |
| **B8** | 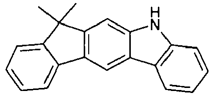 | 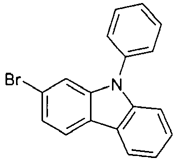 | 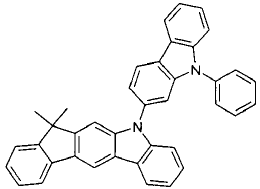 | 53 |
| | WO 2010/136109 | CAS 94994-62-4 | | |
| **B9** | 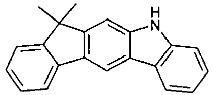 | 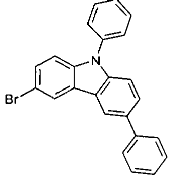 | 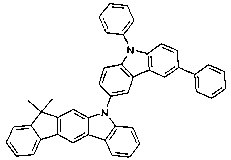 | 45 |
| | WO 2010/136109 | CAS 1160294-85-8 | | |
| **B10** | | 2.2 Äquivalente | 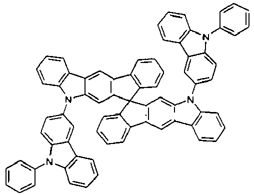 | 64 |
| | 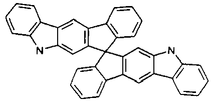 | 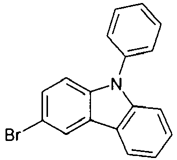 | | |
| | WO 2010/136109 | CAS 1153-85-1 | | |
| **B11** | 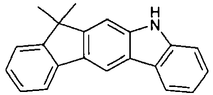 | 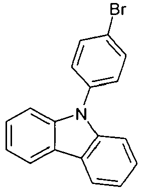 | 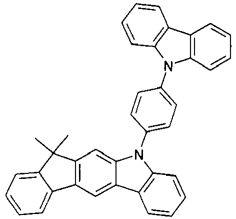 | 61 |
| | WO 2010/136109 | CAS 57102-42-8 | nicht erfindungsgemäß | |
| **B12** | 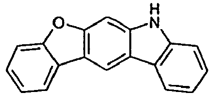 | 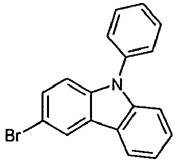 | 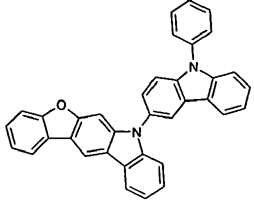 | 57 |
| | CAS 1246308-83-7 | CAS 1153-85-1 | | |
| **B13** | 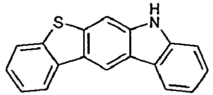 | 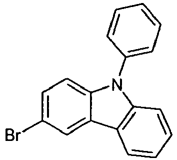 | 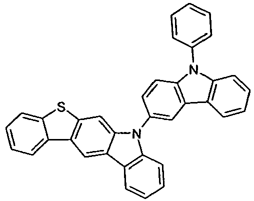 | 48 |
| | CAS 1255309-04-6 | CAS 1153-85-1 | | |
| **B14** | 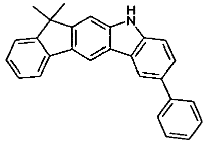 | 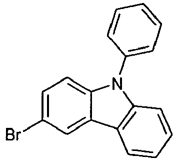 | 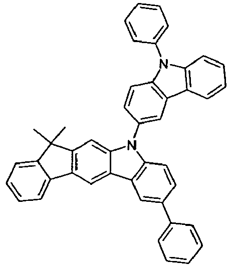 | 78 |
| | WO 2010/136109 | CAS 1153-85-1 | | |
| **B15** | 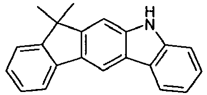 | 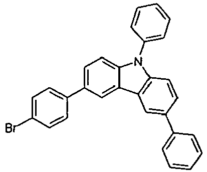 | 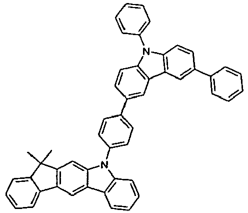 | 58 |
| | WO 2010/136109 | CAS 1186644-47-2 | | |
| **B16** | 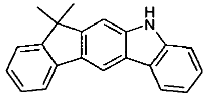 | 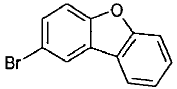 | 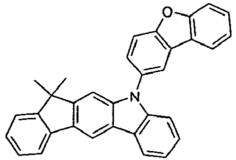 | 54 |
| | WO 2010/136109 | CAS 86-76-0 | | |
| **B17** | 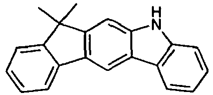 | 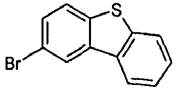 | 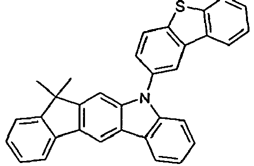 | 45 |
| | WO 2010/136109 | CAS 22439-61-8 | | |
| **B18** | 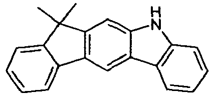 | 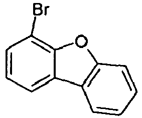 | 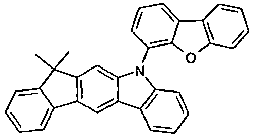 | 57 |
| | WO 2010/136109 | CAS 89827-45-2 | | |
| **B19** | 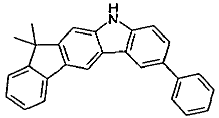 | 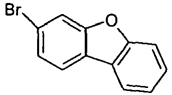 | 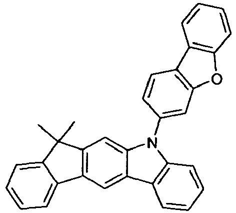 | 51 |
| | WO 2010/136109 | CAS 26608-06-0 | | |
| **B20** | 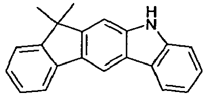 | 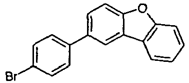 | 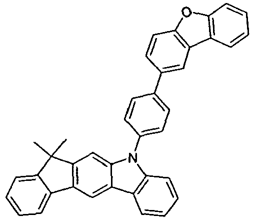 | 54 |
| | WO 2010/136109 | CAS 955959-86-1 | | |
| **B21** | 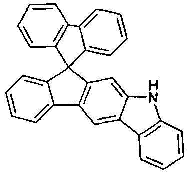 | 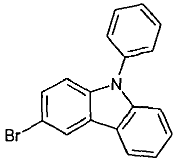 | 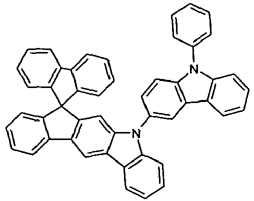 | 62 |
| | WO 2010/136109 | CAS 1153-85-1 | | |
| **B22** | 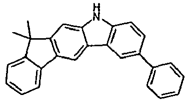 | 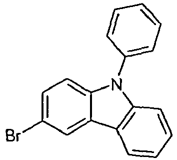 | 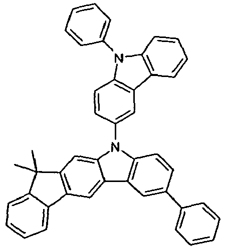 | 43 |
| | WO 2010/136109 | CAS 1153-85-1 | | |
| **B23** | 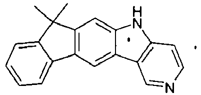 | 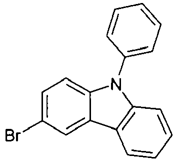 | 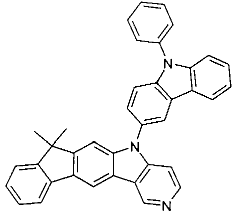 | 54 |
| | WO 2010/136109 | CAS 1153-85-1 | | |
| **B24** | 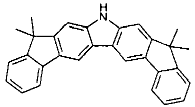 | 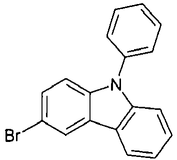 | 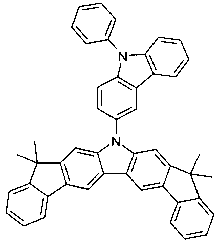 | 56 |
| | WO 2010/136109 | CAS 1153-85-1 | | |
| **B25** | 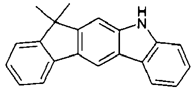 | 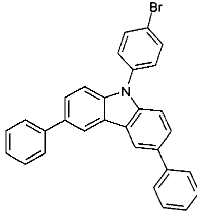 | 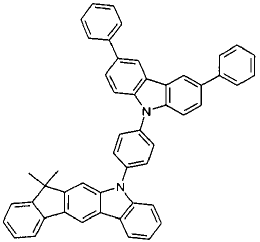 | 54 |
| | WO 2010/136109 | CAS 607739-92-4 | nicht erfindungsgemäß | |
| **B26** | 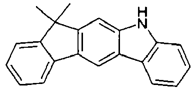 | 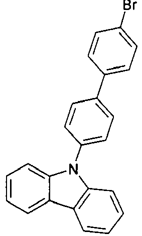 | 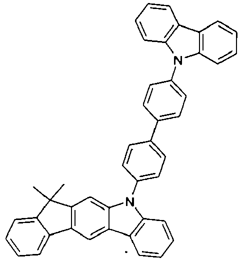 | 53 |
| | WO 2010/136109 | CAS 212385-73-4 | nicht erfindungsgemäß | |
| | | 2.2 Äquivalente | 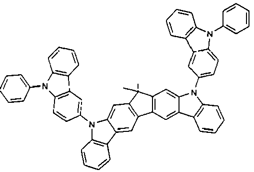 | |
| **B27** | 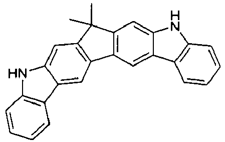 | 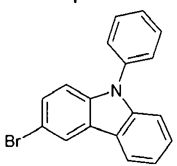 | | 45 |
| | WO 2010/136109 | CAS 1153-85-1 | | |

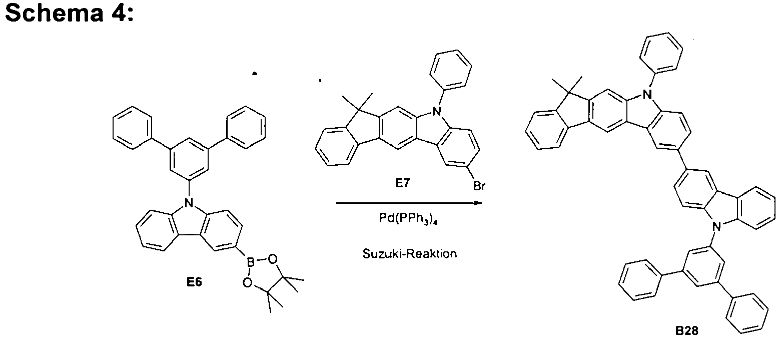

### B28: 12,12-Dimethyl-10-phenyl-7-(9-[1,1';3',1"]terphenyl-5'-yl-9H-carbazol-3-yl)-10,12-dihydro-10-aza-indeno[2,1-b]fluoren (nicht erfindungsgemäß)

In 250 ml Aceton werden 20 g (38 mmol) 9-[1,1';3',1"]-Terphenyl-5'-yl-3-(4,4,5,5-tetramethyl-[1,3,2]dioxaborolan-2-yl)-9H-carbazol **S5** und 17 g (38 mmol, 1 eq) 7-Brom-12,12-dimethyl-10-phenyl-10,12-dihydro-10-aza-indeno-[2,1-b]fluoren (WO 2010/136109) vorgelegt und mit 62 ml (84 mmol, 2.2 eq) Tetraethylammoniumhydroxid (20%-ige Lösung in Wasser) versetzt. Das Reaktionsgemisch wird für 30 Minuten mit Stickstoff entgast und anschließend werden 0.88 g (0.76 mmol, 0.02 eq) Tetrakis(triphenylphosphin)-palladium(0) zugegeben und über Nacht bei 50 °C gerührt. Der ausgefallene Feststoff wird abgesaugt und mittels Heißextraktion, mehrfacher Umkristallisation aus Heptan/Toluol und abschließender Sublimation aufgereinigt. Es werden 14 g (19 mmol, 51%) des Produktes mit einer HPLC-Reinheit >99.9% erhalten werden.

### Analog werden folgende Verbindungen hergestellt:

| **Bsp.** | **E6** | **E7** | **Produkt** | **Ausbeute [%]** |
|---|---|---|---|---|
| **B29** | 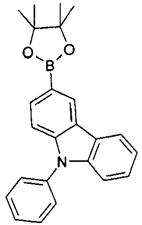 | 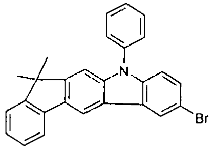 | 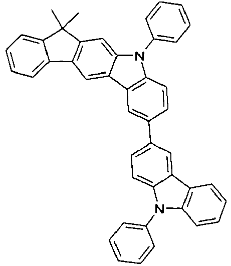 | **63** |
| | **S6** | WO 2010/136109 | nicht erfindungsgemäß | |
| | 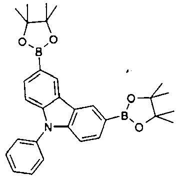 | 2 Äquivalente | 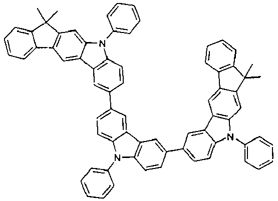 | |
| **B30** | | 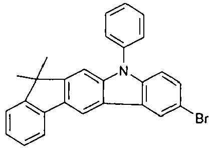 | | 53 |
| | **S7** | WO 2010/136109 | nicht erfindungsgemäß | |

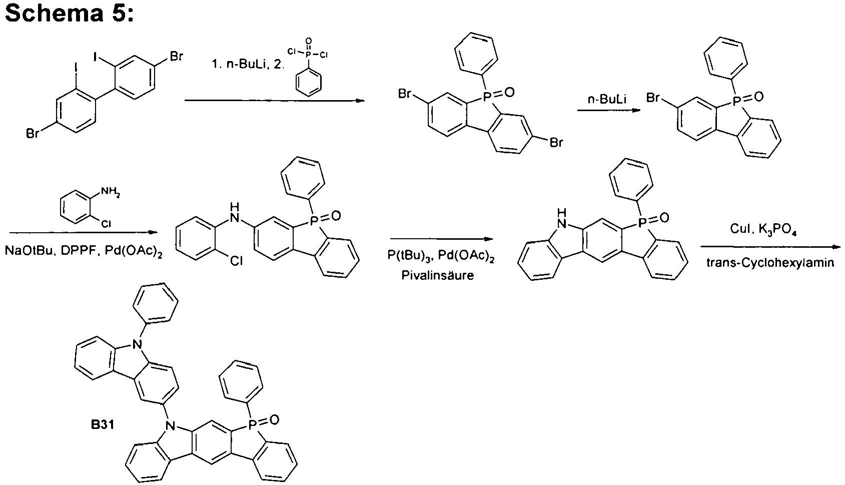

### 3,7-Dibrom-5-phenyl-dibenzophosphol-5-oxid:

Zu einer Lösung aus 30 g (53 mmol) 4,4'-Dibrom-2,2'-diiod-biphenyl in 500 ml trockenem THF werden 66 ml (106 mmol, 2.0 eq) n-Butyllithium (1.6 M in Hexan) bei -78°C gegeben und bei dieser Temperatur für 30 Minuten gerührt. Anschließend werden 11 g (56 mmol, 1.06 eq) Dichlorophenylphosphinoxid zugetropft und das Reaktionsgemisch nach beendeter Reaktion auf Raumtemperatur erwärmt. Nach Hydrolyse mit Wasser wird die organische Phase mit Ether extrahiert und die vereinten organischen Phasen über Natriumsulfat getrocknet. Das Lösungsmittel wird am Rotationsverdampfer entfernt und das erhaltene Rohprodukt säulenchromatographisch aufgereinigt (Heptan/Ethylacetat 6:1). Es werden 20 g (45 mmol, 85%) des Produkts erhalten.

### 3-Brom-5-phenyl-dibenzophosphol-5-oxid:

20 g (45 mmol) 3,7-Dibrom-5-phenyl-dibenzophosphol-5-oxid in 400 ml trockenem THF werden auf -78°C gekühlt und bei dieser Temperatur langsam mit 28 ml (45 mmol, 1.0 eq) n-Buthyllithium (1.6 M in Hexan) versetzt. Nach 1 h wird das Gemisch langsam auf Raumtemperatur erwärmt, 50 ml 1 M HCl zugegeben und für weitere 2 h gerührt. Anschließend wird mit Ethylacetat extrahiert, mit Wasser gewaschen und die vereinten organischen Phasen über Natriumsulfat getrocknet. Die Lösungsmittel werden am Rotationsvedampfer entfernt und das erhaltene Produkt ohne weitere Aufreinigungsschritte verwendet. Es werden 16 g (43 mmol, 96 %) des Monobromids erhalten.

### (2-Chlor-phenyl)-(5-oxo-5-phenyl-5H-5lambda*5*-dibenzophosphol-3-yl)-amin:

In 400 ml Toluol werden 16 g (43 mmol) 3-Brom-5-phenyl-dibenzophosphol-5-oxid zusammen mit 6.6 ml (52 mmol, 1.2 eq) 2-Chloranilin und 11 g (112 mmol, 2.6 eq) Natrium-tert-butylat vorgelegt und mit 480 mg (0.86 mmol, 0.2 eq) DPPF und 97 mg (0.43 mmol, 0.01 eq) Palladiumacetat versetzt. Das Reaktionsgemisch wird über Nacht unter Rückfluss erhitzt und nach beendeter Reaktion mit 200 ml Wasser versetzt. Die Phasen werden getrennt und die wässrige Phase mit Toluol extrahiert. Die vereinten organischen Phasen werden über Natriumsulfat getrocknet und über Alulminiumoxid filtriert. Das Lösungsmittel wird im Vakuum entfernt und der erhaltene Rückstand duch Säulenchromatographie gereinigt (Heptan/Ethylacetat 5:1). Es werden 16 g (39 mmol, 91 %) des Produkts erhalten.

### 12-Phenyl-10H-10-aza-12-phospha-indeno[2,1-b]fluorene 12-oxid:

16 g (39 mmol) (2-Chlorphenyl)-(5-oxo-5-phenyl-5H-5lambda*5*-dibenzophosphol-3-yl)-amin und 14 g (100 mmol, 2.6 eq) Kaliumcarbonat werden in 250 ml NMP vorgelegt und mit 1.4 g (13 mmol, 0.34 eq) Pivalinsäure versetzt. Anschließend werden 3.1 ml einer 1M Tri-tert-Butylphosphin-Lösung in Toluol (3.1 mmol, 0.08 eq) und 440 mg (2.0 mmol, 0.05 eq) Palladiumacetat zugegeben und das Reaktionsgemisch über Nacht auf 130 °C Innentemperatur erhitzt. Der Ansatz wird auf Raumtemperatur abgekühlt und 300 ml Toluol und 100 ml Wasser zugegeben. Die wässrige Phase wird dreimal mit Toluol extrahiert, die vereinten organischen Phasen ebenfalls dreimal mit Wasser gewaschen und abschließend über Natriumsulfat getrocknet. Nach Entfernen des Lösungsmittels wird das erhaltene Rohprodukt durch Säulenchromatographie aufgereinigt. Es werden 13 g (35 mmol, 89 %) des Produkts erhalten.

### B31: 12-Phenyl-10-(9-phenyl-9H-carbazol-3-yl)-10H-10-aza-12-phospha-indeno[2,1-b]fluoren 12-oxid:

Das Experiment wird analog **B1** durchgeführt. Es werden 14 g (23 mmol, 67 %) des Zielproduktes **B31** erhalten.

### Teil C: Vergleich der thermischen Stabilität

Schmilzt man 100 mg der Verbindung ICvCbz1 in einer Glasampulle unter Vakuum (Druck ca. 10⁻² mbar) ab und lagert diese bei 310 °C für 14 Tage in einem Ofen, so verändert sich die Reinheit gemäß HPLC von 99.7% auf 89.2%. Mit der Verbindung B29 ändert sich die Reinheit gemäß HPLC bei gleicher Vorgehensweise von 99.8% auf 99.6%, d. h. es entstehen unter gleicher thermischer Belastung weit weniger Zersetzungsprodukte. Dies ist ein wesentlicher technischer Vorteil, da die Materialien in der technischen Produktion organischer Elektrolumineszenzvorrichtungen lange Zeit hohen Temperaturen ausgesetzt sind.

### Teil D: Organische Elektrolumineszenzvorrichtungen

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen V1 bis E17 (siehe Tabellen 1 und 2) werden die Daten verschiedener OLEDs vorgestellt. Glasplättchen, die mit strukturiertem ITO (Indium Zinn Oxid) der Dicke 50 nm beschichtet sind werden zur verbesserten Prozessierung mit 20 nm PEDOT:PSS beschichtet (Poly(3,4-ethylendioxythiophen)poly(styrolsulfonat), bezogen als CLEVIOS™ P VP Al 4083 von Heraeus Precious Metals GmbH Deutschland, aus wässriger Lösung aufgeschleudert). Diese beschichteten Glasplättchen bilden die Substrate, auf welche die OLEDs aufgebracht werden. Die OLEDs haben folgenden Schichtaufbau: Substrat / Lochtransportschicht (HTL) / Zwischenschicht (IL) / Elektronenblockierschicht (EBL) / Emissionsschicht (EML) / Lochblockierschicht (HBL) / Elektronentransportschicht (ETL) und abschließend eine 100 nm dicke Aluminiumkathode. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC2:TEG1 (30%:60%:10%) bedeutet hierbei, dass das Material IC1 in einem Volumenanteil von 30%, IC2 in einem Volumenanteil von 60% und TEG1 in einem Volumenanteil von 10% in der Schicht vorliegt. Analoges gilt für die Elektronentransportschicht.

Die OLEDs werden standardmäßig charakterisiert. Hierfür werden die Elektrolumineszenzspektren, die Stromeffizienz (gemessen in cd/A), die Leistungseffizienz (gemessen in lm/W/) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m². Als Lebensdauer LD wird die Zeit definiert, nach der die Leuchtdichte bei Betrieb mit konstantem Strom von der Startleuchtdichte L0 auf einen gewissen Anteil L1 absinkt. Eine Angabe von L0 = 10000 cd/m² und L1 = 70% in Tabelle 2 bedeutet, dass die in Spalte LD angegebene Lebensdauer der Zeit entspricht, nach der die Anfangsleuchtdichte von 10000 cd/m² auf 7000 cd/m² absinkt.

Die Daten der verschiedenen OLEDs sind in Tabelle 2 zusammengefasst. Die Beispiel V1-V8 sind Vergleichsbeispiele gemäß dem Stand der Technik, die Beispiele E1-E17 zeigen Daten von OLEDs mit erfindungsgemäßen Materialien.

Im Folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt. Wie sich der Tabelle entnehmen lässt, werden auch bei Verwendung der nicht näher ausgeführten erfindungsgemäßen Verbindungen deutliche Verbesserungen gegenüber dem Stand der Technik erzielt.

### Verwendung von erfindungsgemäßen Verbindungen als Komponente eines Mixed-Matrix Systems

In den folgenden Beispielen werden Daten von OLEDs gezeigt, bei denen das Mischungsverhältnis so gewählt ist, dass eine maximale Lebensdauer erhalten wird.

Vergleicht man die Beispiele V2 und E1, so sieht man, dass die erfindungsgemäße Verbindung B2, welche einen Carbazol-Substituenten am Stickstoff trägt, gegenüber der Verbindung IC2 gemäß dem Stand der Technik mit einem Terphenyl-Substituenten deutliche bessere Werte liefert. Die Leistungseffizienz wird mit B2 um fast 15%, die Lebensdauer um etwa 20% verbessert.

Ebenso erhält man eine deutliche Verbesserung beim Austausch eines Indenocarbazol- gegen einen Carbazol-Substituenten (Beispiele V1 und E2). In diesem Fall erhöht sich die Lebensdauer fast auf das doppelte, die Verbesserung in der Leistungseffizienz ist mit 20% ebenfalls sehr hoch. Auch gegenüber dem Bis-Carbazol BCbz1 erhält man mit B29 eine deutlich bessere Lebensdauer und Leistungseffizienz (Beispiele V3 und E2).

Bei Austausch eines verbrückten Carbazols gegen ein unverbrücktes Carbazol (Beispiele V5 und E2) steigert sich die Lebensdauer um 60%. Zwar erhält man mit der Verbindung ICvCbz1 gemäß dem Stand der Technik eine etwas bessere Quanteneffizienz als mit der erfindungsgemäßen Verbindung B29, aufgrund der besseren Spannung ergibt sich aber die gleiche Leistungseffizienz.

Reduziert man bei OLEDs enthaltend Materialien gemäß dem Stand der Technik die Emitterkonzentration auf deutlich unter 10%, so verringern sich Effizienz und Lebensdauer signifikant. Bei Einsatz der Verbindung IC2 z.B. verringert sich die externe Quanteneffizienz um fast 10% bei Verringerung der Emitterkonzentration von 10% auf 4%. Viel signifikanter ist die Verschlechterung der Lebensdauer um einen Faktor von mehr als 1.5 (Beispiele V7 und V8). Mit erfindungsgemäßen Materialien hingegen ist keine Leistungseinbuße (Beispiel E1 verglichen mit E12) oder sogar eine leichte Verbesserung zu beobachten (Beispiel E2 verglichen mit E13-E15).

Die erfindungsgemäßen Materialien ergeben bei Einsatz als Matrixmaterialien in phosphoreszierenden OLEDs somit wesentliche Verbesserungen gegenüber dem Stand der Technik in einigen oder allen Parametern. Weiterhin lassen sich mit erfindungsgemäßen Materialien OLEDs mit geringen Emitterkonzentrationen ohne Leistungseinbußen realisieren.

| Tabelle 1: Aufbau der OLEDs | | | | | | |
|---|---|---|---|---|---|---|
| Bsp. | HTL Dicke | IL Dicke | EBL Dicke | EML Dicke | HBL Dicke | ETL Dicke |
| V1 | SpA1 | HATCN | SpMA1 | IC1:BIC1:TEG1 | IC1 | ST1:LiQ(50%:50%) |
| | 70nm | 5nm | 90nm | (60%:30%:10%) 30nm | 10nm | 30nm |
| V2 | SpA1 | HATCN | SpMA1 | IC1:IC2:TEG1 | IC1 | ST1:LiQ(50%:50%) |
| | 70nm | 5nm | 90nm | (30%:60%:10%) 30nm | 10nm | 30nm |
| V3 | SpA1 | HATCN | SpMA1 | IC1:BCbz1:TEG1 | IC1 | ST1:LiQ (50%:50%) |
| | 70nm | 5nm | 90nm | (30%:60%:10%) 30nm | 10nm | 30nm |
| V4 | SpA1 | HATCN | SpMA1 | IC1:IC3:TEG1 | IC1 | ST1:LiQ(50%:50%) |
| | 70nm | 5nm | 90nm | (70%:20%:10%) 30nm | 10nm | 30nm |
| V5 | SpA1 | HATCN | SpMA1 | IC1:ICvCbz1:TEG1 | IC1 | ST1:LiQ(50%:50%) |
| | 70nm | 5nm | 90nm | (60%:30%:10%) 30nm | 10nm | 30nm |
| V6 | SpA1 | HATCN | BPA1 | ST1:BIC2:TEG1 | ST1 | ST1:LiQ(50%:50%) |
| | 70nm | 5nm | 90nm | (30%:60%:10%) 30nm | 10nm | 30nm |
| V7 | SpA1 | HATCN | PA1 | IC1:IC2:TEG1 | ST1 | ST1:LiQ (50%:50%) |
| | 70nm | 5nm | 90nm | (30%:60%:10%) 30nm | 10nm | 30nm |
| V8 | SpA1 | HATCN | PA1 | IC1:IC2:TEG1 | ST1 | ST1:LiQ (50%:50%) |
| | 70nm | 5nm | 90nm | (32%:64%:4%) 30nm | 10nm | 30nm |
| E1 | SpA1 | HATCN | SpMA1 | IC1:B2:TEG1 | IC1 | ST1:LiQ (50%:50%) |
| | 70nm | 5nm | 90nm | (30%:60%:10%) 30nm | 10nm | 30nm |
| E2* | SpA1 | HATCN | SpMA1 | IC1:B29:TEG1 | IC1 | ST1:LiQ (50%:50%) |
| | 70nm | 5nm | 90nm | (55%:35%:10%) 30nm | 10nm | 30nm |
| E3 * | SpA1 | HATCN | SpMA1 | IC1:B30:TEG1 | IC1 | ST1:LiQ (50%:50%) |
| | 70nm | 5nm | 90nm | (65%:25%:10%) 30nm | 10nm | 30nm |
| E4* | SpA1 | HATCN | BPA1 | ST1:B26:TEG1 | ST1 | ST1:LiQ (50%:50%) |
| | 70nm | 5nm | 90nm | (30%:60%:10%) 30nm | 10nm | 30nm |
| E5 | SpA1 | HATCN | SpMA1 | IC1:B27:TEG1 | IC1 | ST1:LiQ (50%:50%) |
| | 70nm | 5nm | 90nm | (65%:25%:10%) 30nm | 10nm | 30nm |
| E6 | SpA1 | HATCN | SpMA1 | IC1:B5:TEG1 | IC1 | ST1:LiQ (50%:50%) |
| | 70nm | 5nm | 90nm | (30%:60%:10%) 30nm | 10nm | 30nm |
| E7 | SpA1 | HATCN | SpMA1 | IC1:B8:TEG1 | IC1 | ST1:LiQ (50%:50%) |
| | 70nm | 5nm | 90nm | (30%:60%:10%) 30nm | 10nm | 30nm |
| E8 | SpA1 | HATCN | SpMA1 | IC1:B14:TEG1 | IC1 | ST1:LiQ (50%:50%) |
| | 70nm | 5nm | 90nm | (30%:60%:10%) 30nm | 10nm | 30nm |
| E9 | SpA1 | HATCN | SpMA1 | ST1:B12:TEG1 | ST1 | ST1:LiQ (50%:50%) |
| | 70nm | 5nm | 90nm | (25%:65%:10%) 30nm | 10nm | 30nm |
| E10 | SpA1 | HATCN | SpMA1 | IC1:B24:TEG1 | IC1 | ST1:LiQ(50%:50%) |
| | 70nm | 5nm | 90nm | (50%:50%:10%) 30nm | 10nm | 30nm |
| E11 | SpA1 | HATCN | SpMA1 | ST1:B18:TEG1 | ST1 | ST1:LiQ (50%:50%) |
| | 70nm | 5nm | 90nm | (30%:60%:10%) 30nm | 10nm | 30nm |
| E12 | SpA1 | HATCN | SpMA1 | IC1:B2:TEG1 | IC1 | ST1:LiQ (50%:50%) |
| | 70nm | 5nm | 90nm | (32%:64%:4%) 30nm | 10nm | 30nm |
| E13* | SpA1 | HATCN | SpMA1 | IC1:B29:TEG1 | IC1 | ST1:LiQ (50%:50%) |
| | 70nm | 5nm | 90nm | (60%:39%:1%) 30nm | 10nm | 30nm |
| E14* | SpA1 | HATCN | SpMA1 | IC1:B29:TEG1 | IC1 | ST1:LiQ (50%:50%) |
| | 70nm | 5nm | 90nm | (59%:37%:4%) 30nm | 10nm | 30nm |
| E15* | SpA1 | HATCN | SpMA1 | IC1:B29:TEG1 | IC1 | ST1:LiQ (50%:50%) |
| | 70nm | 5nm | 90nm | (57%:36%:7%) 30nm | 10nm | 30nm |
| E16 | SpA1 | HATCN | SpMA1 | IC1:B10:TEG1 | IC1 | ST1:LiQ (50%:50%) |
| | 70nm | 5nm | 90nm | (45%:45%:10%) 30nm | 10nm | 30nm |
| E17 | SpA1 | HATCN | SpMA1 | IC1:B23:TEG1 | IC1 | ST1:LiQ (50%:50%) |
| | 70nm | 5nm | 90nm | (30%:60%:10%) 30nm | 10nm | 30nm |

| | | | | | | |
|---|---|---|---|---|---|---|
| * nicht erfindungsgemäß | | | | | | |

**Tabelle 2: Daten der OLEDs**

| Bsp. | U1000 (V) | SE1000 (cd/A) | LE1000 (lm/W) | EQE 1000 | CIE x/y bei 1000 cd/m² | L0 | L1 % | LD (h) |
|---|---|---|---|---|---|---|---|---|
| V1 | 3.6 | 51 | 44 | 14.1% | 0.34/0.62 | 10000 cd/m² | 70 | 240 |
| V2 | 3.6 | 52 | 46 | 14.5% | 0.33/0.62 | 10000 cd/m² | 70 | 270 |
| V3 | 3.6 | 56 | 49 | 15.6% | 0.34/0.62 | 10000 cd/m² | 70 | 220 |
| V4 | 3.2 | 54 | 54 | 15.1% | 0.33/0.62 | 10000 cd/m² | 80 | 190 |
| V5 | 3.5 | 58 | 52 | 16.1% | 0.32/0.63 | 10000 cd/m² | 70 | 280 |
| V6 | 3.8 | 51 | 43 | 14.3% | 0.33/0.63 | 10000 cd/m² | 70 | 210 |
| V7 | 3.5 | 53 | 48 | 14.7% | 0.33/0.62 | 10000 cd/m² | 70 | 240 |
| V8 | 3.5 | 49 | 44 | 13.6% | 0.33/0.63 | 10000 cd/m² | 70 | 140 |
| E1 | 3.4 | 56 | 52 | 15.6% | 0.33/0.63 | 10000 cd/m² | 70 | 320 |
| E2 * | 3.3 | 55 | 52 | 15.5% | 0.34/0.62 | 10000 cd/m² | 70 | 450 |
| E3 * | 3.4 | 57 | 54 | 16.0% | 0.33/0.62 | 10000 cd/m² | 70 | 410 |
| E4 * | 3.4 | 55 | 51 | 15.2% | 0.33/0.63 | 10000 cd/m² | 70 | 270 |
| E5 | 3.2 | 57 | 56 | 15.9% | 0.33/0.63 | 10000 cd/m² | 80 | 250 |
| E6 | 3.5 | 53 | 48 | 14.8% | 0.33/0.63 | 10000 cd/m² | 70 | 290 |
| E7 | 3.5 | 55 | 49 | 15.2% | 0.33/0.62 | 10000 cd/m² | 70 | 300 |
| E8 | 3.6 | 55 | 49 | 15.4% | 0.33/0.62 | 10000 cd/m² | 70 | 330 |
| E9 | 3.6 | 53 | 46 | 14.6% | 0.33/0.63 | 10000 cd/m² | 70 | 250 |
| E10 | 3.4 | 58 | 54 | 16.2% | 0.33/0.63 | 10000 cd/m² | 70 | 330 |
| E11 | 3.5 | 55 | 49 | 15.3% | 0.32/0.62 | 10000 cd/m² | 70 | 270 |
| E12 | 3.3 | 55 | 52 | 15.3% | 0.33/0.63 | 10000 cd/m² | 70 | 310 |
| E13 * | 3.3 | 61 | 58 | 16.9% | 0.33/0.62 | 10000 cd/m² | 70 | 510 |
| E14 * | 3.2 | 63 | 62 | 17.7% | 0.33/0.63 | 10000 cd/m² | 70 | 460 |
| E15 * | 3.3 | 59 | 57 | 16.6% | 0.33/0.62 | 10000 cd/m² | 70 | 480 |
| E16 | 3.5 | 54 | 49 | 15.1% | 0.33/0.63 | 10000 cd/m² | 70 | 290 |
| E17 | 3.4 | 57 | 52 | 15.8% | 0.33/0.62 | 10000 cd/m² | 70 | 310 |

| | | | | | | | | |
|---|---|---|---|---|---|---|---|---|
| * nicht erfindungsgemäß | | | | | | | | |

**Tabelle 3: Strukturformeln der Materialien für die OLEDs**

| | |
|---|---|
| 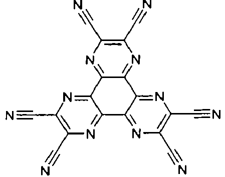 | 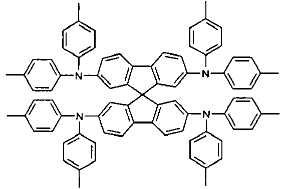 |
| HATCN | SpA1 |
| 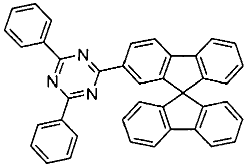 | 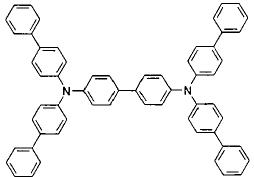 |
| ST1 | BPA1 |
| 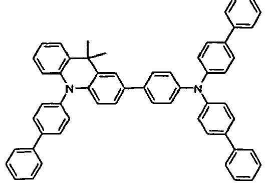 | 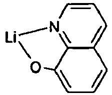 |
| PA1 | LiQ |
| 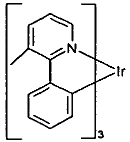 | 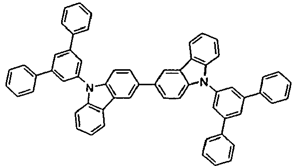 |
| TEG1 | BCbz1 |
| 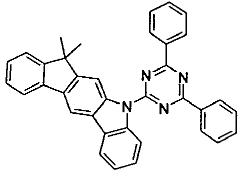 | 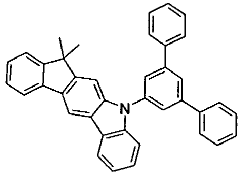 |
| IC1 | IC2 |
| 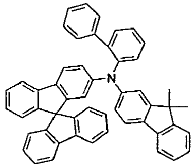 | 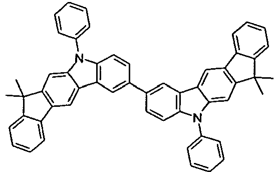 |
| SpMA1 | BIC1 |
| 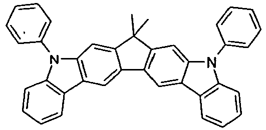 | 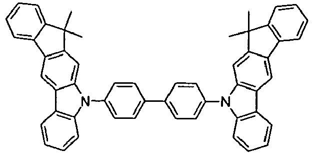 |
| IC3 | BIC2 |
| 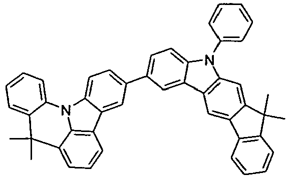 | 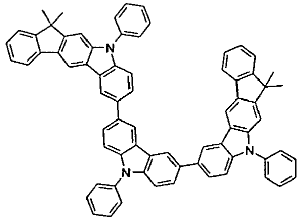 |
| ICvCbz1 | B30 |
| 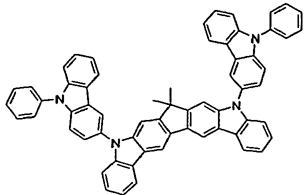 | 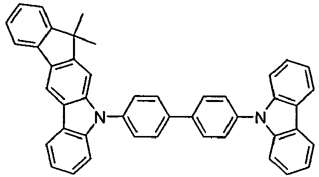 |
| B27 | B26 |
| 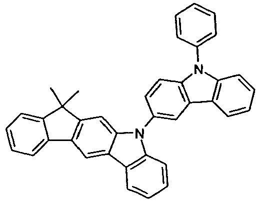 | 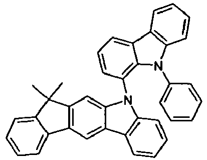 |
| B2 | B5 |
| 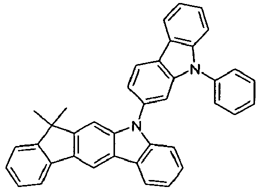 | 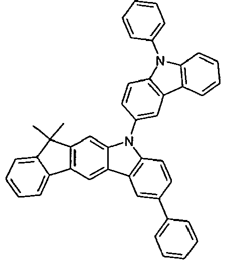 |
| B8 | B14 |
| 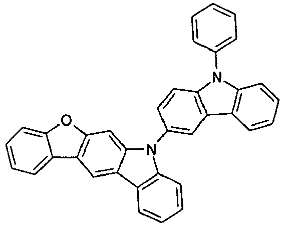 | 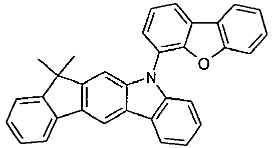 |
| B12 | B18 |
| 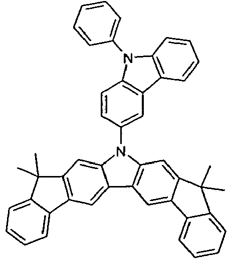 | 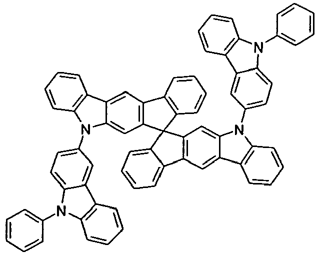 |
| B24 | B10 |
| 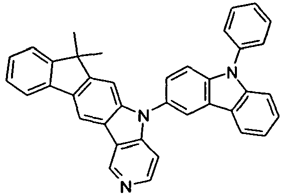 | 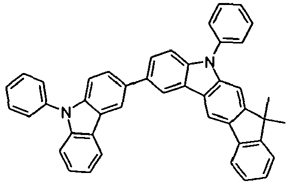 |
| B23 | B29 |

## Patentansprüche

1. Verbindung gemäß Formel (1), 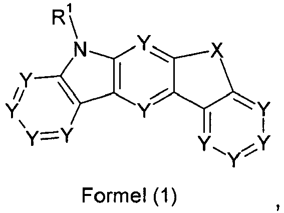 wobei für die verwendeten Symbole gilt:
Y ist bei jedem Auftreten gleich oder verschieden CR oder N;
X ist ausgewählt aus C(R¹)₂, O, S, PR¹, P(=O)R¹ oder BR¹;
R, R¹ ist bei jedem Auftreten gleich oder verschieden H, D, F, Cl, Br, I, N(R²)₂, N(Ar)₂, C(=O)Ar, P(=O)Ar₂, S(=O)Ar, S(=O)₂Ar, CR²=CR²Ar, CN, NO₂, Si(R²)₃, B(OR²)₂, OSO₂R², eine gerad-kettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, C=O, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxy-gruppe mit 5 bis 40 aromatischen Ringatomen, die durch einen oder mehrere Reste R² substituiert sein kann, oder eine Aralkyl- oder Heteroaralkylgruppe mit 5 bis 40 aromatischen Ringatomen, die durch einen oder mehrere Reste R² substituiert sein kann, oder eine Kombination dieser Systeme; dabei können zwei oder mehrere Substituenten R zusammen mit den Atomen, an die sie gebunden sind, bzw. zwei Substituenten R¹ zusammen mit dem Atom, an das sie gebunden sind, auch miteinander ein mono- oder polycyclisches, aliphatisches oder aromatisches Ringsystem bilden;
Ar ist bei jedem Auftreten gleich oder verschieden ein aromatisches oder heteroaromatisches Ringsystem mit 5 bis 40 aromatischen Ringatomen, das mit einem oder mehreren Resten R³ substituiert sein kann;
R² ist bei jedem Auftreten gleich oder verschieden H, D, F, Cl, Br, I, N(R³)₂, N(Ar)₂, C(=O)Ar, P(=O)Ar₂, S(=O)Ar, S(=O)₂Ar, CR³=CR³Ar, CN, NO₂, Si(R³)₃, B(OR³)₂, OSO₂R³, eine gerad-kettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R³ substituiert sein kann, wobei eine oder mehrere nicht benachbarte CH₂-Gruppen durch R³C=CR³, C≡C, Si(R³)₂, C=O, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Aryloxy- oder Heteroaryloxygruppe mit 5 bis 40 aromatischen Ringatomen, die durch einen oder mehrere Reste R³ substituiert sein kann, oder eine Aralkyl- oder Heteroaralkylgruppe mit 5 bis 40 aromatischen Ringatomen, die durch einen oder mehrere Reste R³ substituiert sein kann, oder eine Kombination dieser Systeme;
R³ ist bei jedem Auftreten gleich oder verschieden H, D oder ein aliphatischer Kohlenwasserstoffrest mit 1 bis 20 C-Atomen oder eine Aryl- oder Heteroarylgruppe mit 5 bis 40 Ringatomen oder eine Kombination dieser Gruppen;
mit der Maßgabe, dass, wenn eine oder mehrere der Gruppen R, R¹, R², R³, Ar oder Ar¹ Heteroarylgruppen enthalten, welche nicht den Formeln (2) oder (3) entsprechen, es sich dabei nicht um elektronenarme Heteroarylgruppen handelt, wobei eine elektronenarme Heteroarylgruppe eine 5-Ring-Heteroarylgruppe mit mindestens zwei Heteroatomen oder eine 6-Ring-Heteroarylgruppe mit mindestens einem Heteroatom ist und an diese Gruppen noch weitere 6-Ring-Aryl- oder 6-Ring-Heteroarylgruppen ankondensiert sein können;
**dadurch gekennzeichnet, dass** mindestens eine Gruppe R vorhanden ist, die gleich oder verschieden bei jedem Auftreten für eine Gruppe der folgenden Formel (2) steht, 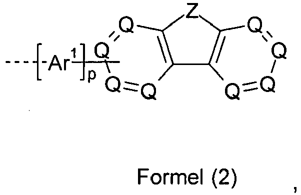 wobei die gestrichelte Bindung die Verknüpfung der Gruppe der Formel (2) andeutet, R² die oben genannten Bedeutungen aufweist und weiterhin gilt:
Q ist C, wenn die Gruppe der Formel (2) über diese Gruppe mit Ar¹ bzw. mit dem restlichen Molekül verknüpft ist; bzw. ist gleich oder verschieden bei jedem Auftreten CR² oder N in den sonstigen Fällen;
Z ist NR²;
Ar¹ ist ein bivalentes aromatisches oder heteroaromatisches Ring-system mit 5 bis 40 aromatischen Ringatomen, welches durch einen oder mehrere Reste R² substituiert sein kann;
p ist 0 oder 1;
und/oder dass mindestens eine Gruppe R¹ vorhanden ist, die für eine Gruppe der folgenden Formel (3) steht, 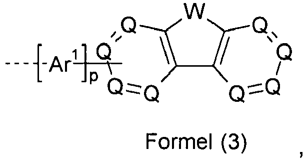 wobei die gestrichelte Bindung die Verknüpfung der Gruppe der Formel (3) andeutet, R², Ar¹, Q und p die oben genannten Bedeutungen aufweisen und weiterhin gilt:
W ist NR², O oder S;
dabei sind Verbindungen der folgenden allgemeinen Formel von der Erfindung ausgenommen: 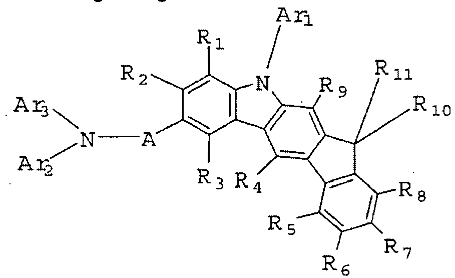 wobei für die verwendeten Symbole gilt:
A ist eine divalente substituierte oder unsubstituierte aromatische Kohlenwasserstoffgruppe, ein divalenter substituierter oder unsubstituierter aromatischer heterocyclischer Ring oder eine divalente substituierte oder unsubstituierte kondensierte polycyclische aromatische Gruppe;
Ar₁, Ar₂, Ar₃ sind gleich oder verschieden und stehen für eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe, wobei A und Ar₂ über eine Einfachbindung aneinander gebunden sind und so einen Ring bilden;
R₁ bis R₉ sind gleich oder verschieden und stehen für ein Wasserstoffatom, ein Deuteriumatom, ein Fluoratom, ein Chloratom, Cyano, Nitro, lineares oder verzweigtes Alkyl mit 1 bis 6 Kohlenstoffatomen, das einen Substituenten tragen kann, Cycloalkyl mit 5 bis 10 Kohlenstoffatomen, das einen Substituierten tragen kann, lineares oder verzweigtes Alkenyl mit 2 bis 6 Kohlenstoffatomen, das einen Substituenten tragen kann, lineares oder verzweigtes Alkyloxy mit 1 bis 6 Kohlenstoffatomen, das einen Substituenten tragen kann, Cycloalkyloxy mit 5 bis 10 Kohlenstoffatomen, das einen Substituenten tragen kann, eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder substituiertes oder unsubstituiertes aryloxy, welche über eine Einfachbindung, substituiertes oder unsubstituiertes Methylen, ein Sauerstoffatom oder ein Schwefelatom aneinander binden und so einen Ring bilden können; und
R₁₀ und R₁₁ sind gleich oder verschieden und stehen für lineares oder verzweigtes Alkyl mit 1 bis 6 Kohlenstoffatomen, das einen Substituenten tragen kann, Cycloalkyl mit 5 bis 10 Kohlenstoffatomen, das einen Substituenten tragen kann, lineares oder verzweigtes Alkenyl mit 2 bis 6 Kohlenstoffatomen, das einen Substituenten tragen kann, lineares oder verzweigtes Alkyloxy mit 1 bis 6 Kohlenstoffatomen, das einen Substituenten tragen kann, Cycloalkyloxy mit 5 bis 10 Kohlenstoffatomen, das einen Substituenten tragen kann, eine substituierte oder unsubstituierte aromatische Kohlenwasserstoffgruppe, eine substituierte oder unsubstituierte aromatische heterocyclisch Gruppe, eine substituierte oder unsubstituierte kondensierte polycyclische aromatische Gruppe oder substituiertes oder unsubstituiertes Aryloxy, welche über eine Einfachbindung, substituiertes oder unsubstituiertes Methylen, ein Sauerstoffatom oder ein Schwefelatom aneinander binden können und so einen Ring bilden.

2. Verbindung nach Anspruch 1, **dadurch gekennzeichnet, dass** der Rest R¹, der an das Stickstoffatom bindet, für ein aromatisches oder heteroaromatisches Ringsystem mit 5 bis 60 aromatischen Ringatomen, das auch durch einen oder mehrere Reste R² substituiert sein kann, oder für eine Gruppe der Formel (3) steht.

3. Verbindung nach Anspruch 1 oder 2, **dadurch gekennzeichnet, dass** X für C(R¹)₂ steht, wobei R¹ gleich oder verschieden bei jedem Auftreten für eine geradkettige Alkylgruppe mit 1 bis 10 C-Atomen oder eine verzweigte oder cyclische Alkylgruppe mit 3 bis 10 C-Atomen, die jeweils mit einem oder mehreren Resten R² substituiert sein kann, wobei eine oder mehrere nicht benachbarte CH₂-Gruppen durch R²C=CR², C≡C , Si(R²)₂, C=O, O, S oder CONR² ersetzt sein können und wobei ein oder mehrere H-Atome durch D, F oder CN ersetzt sein können, oder für ein aromatisches oder heteroaromatisches Ringsystem mit 5 bis 60 aromatischen Ringatomen, das auch durch einen oder mehrere Reste R² substituiert sein kann, steht; dabei können die Reste R¹ auch miteinander ein aromatisches oder aliphatisches Ringsystem bilden.

4. Verbindung nach einem oder mehreren der Ansprüche 1 bis 3, **dadurch gekennzeichnet, dass** maximal eine Gruppe Y pro Cyclus für N steht und die verbleibenden Gruppen Y gleich oder verschieden bei jedem Auftreten für CR stehen.

5. Verbindung nach einem oder mehreren der Ansprüche 1 bis 4 ausgewählt aus Verbindungen gemäß Formel (5), 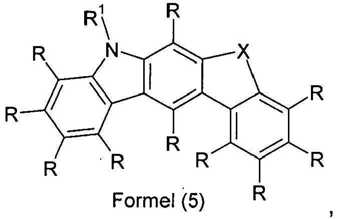 wobei die verwendeten Symbole die in Anspruch 1 genannten Bedeutungen aufweisen.

6. Verbindung nach Anspruch 5, ausgewählt aus den Strukturen der Formeln (6), (7) oder (8), 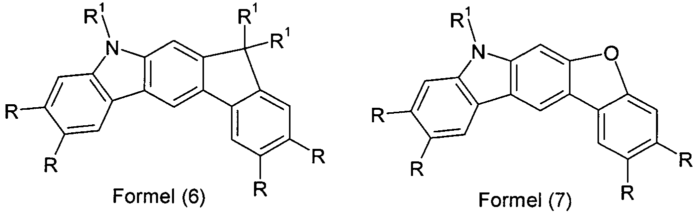 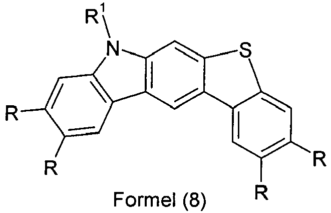 wobei die verwendeten Symbole die in Anspruch 1 genannten Bedeutungen aufweisen.

7. Verbindung nach Anspruch 6, ausgewählt aus den Verbindungen der Formeln (6b), (7b) oder (8b), 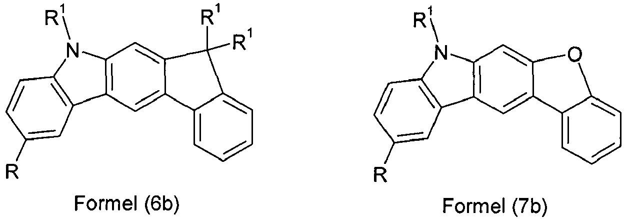 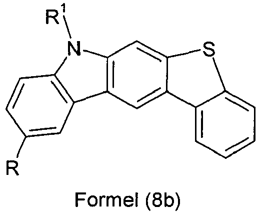 wobei die verwendeten Symbole die in Anspruch 1 genannten Bedeutungen aufweisen.

8. Verbindung nach einem oder mehreren der Ansprüche 1 bis 7, **dadurch gekennzeichnet, dass** die Gruppen der Formeln (2) bis (3) ausgewählt sind aus den Gruppen der Formeln (2a) bis (3a), 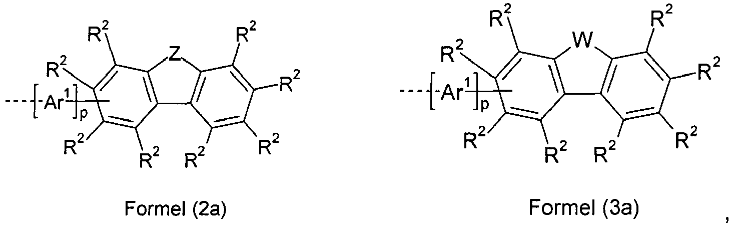 wobei die verwendeten Symbole und Indizes die in Anspruch 1 genannten Bedeutungen aufweisen und in Formel (2a) und (3a) an der Position, an der die Gruppe mit Ar¹ bzw. dem restlichen Molekül verknüpft ist, keine Gruppe R² gebunden ist.

9. Verbindung nach einem oder mehreren der Ansprüche 1 bis 8, **dadurch gekennzeichnet, dass** die Gruppen der Formeln (2) bis (4) ausgewählt sind aus den Strukturen der Formeln (2b), (2c), (2d), (3b), (3c) oder (3d), 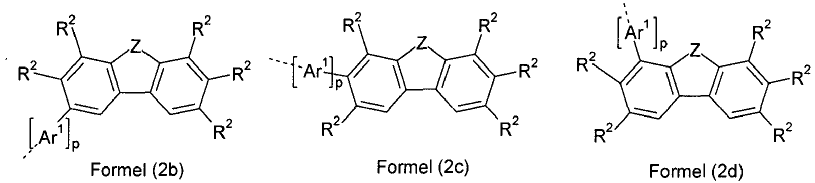 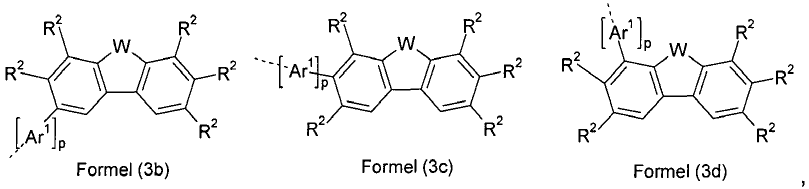 wobei die gestrichelte Bindung die Verknüpfung der Gruppe mit dem restlichen Molekül andeutet und die weiteren verwendeten Symbole und Indizes die in Anspruch 1 genannten Bedeutungen aufweisen.

10. Verbindung nach Anspruch 8 oder 9, **dadurch gekennzeichnet, dass** die Reste R², welche in Formel (2a) bis (3d) an ein Kohlenstoffatom gebunden sind, für H stehen.

11. Verbindung nach einem oder mehreren der Ansprüche 1 bis 10, **dadurch gekennzeichnet, dass** Z bzw. W für NR² steht, wobei R² für ein aromatisches oder heteroaromatisches Ringsystem steht.

12. Verbindung nach einem oder mehreren der Ansprüche 1 bis 11, ausgewählt aus Strukturen der Formel (9), (10), (11) oder (12), 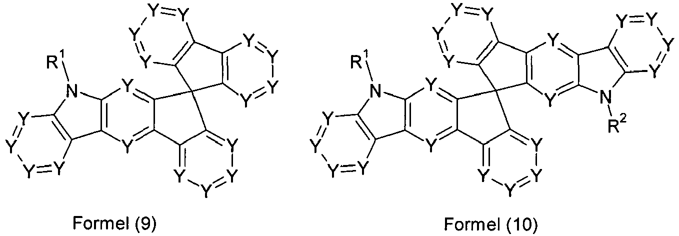 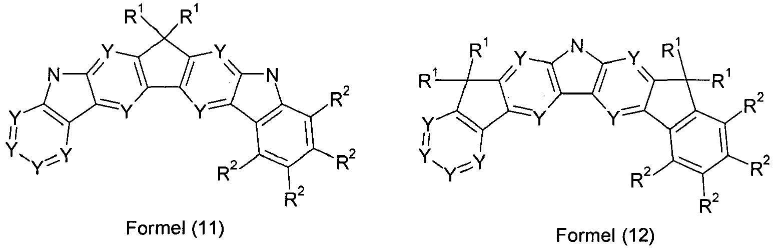 wobei die verwendeten Symbole die in Anspruch 1 genannten Bedeutungen aufweisen.

13. Verfahren zur Herstellung einer Verbindung nach einem oder mehreren der Ansprüche 1 bis 12, **dadurch gekennzeichnet, dass** die Gruppe der Formel (2) oder (3) durch eine Suzuki-Kupplung, eine Ullmann-Kupplung oder durch eine Hartwig-Buchwald-Kupplung eingeführt wird.

14. Mischung enthaltend mindestens eine Verbindung nach einem oder mehreren der Ansprüche 1 bis 12 und mindestens einen fluoreszierenden oder phosphoreszierenden Dotanden.

15. Formulierung, insbesondere eine Lösung, eine Suspension oder eine Miniemulsion, enthaltend mindestens eine Verbindung nach einem oder mehreren der Ansprüche 1 bis 12 oder eine Mischung nach Anspruch 14 und ein oder mehrere Lösemittel.

16. Verwendung einer Verbindung nach einem oder mehreren der Ansprüche 1 bis 12 oder einer Mischung nach Anspruch 14 in einer elektronischen Vorrichtung.

17. Elektronische Vorrichtung, insbesondere ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoff-sensibilisierten Solarzellen, organischen optischen Detektoren, organischen Photorezeptoren, organischen Feld-Quench-Devices, lichtemittierenden elektrochemischen Zellen, organischen Laserdioden und "organic plasmon emitting devices", enthaltend mindestens eine Verbindung nach einem oder mehreren der Ansprüche 1 bis 12 oder eine Mischung nach Anspruch 14.

18. Elektronische Vorrichtung nach Anspruch 17, **dadurch gekennzeichnet, dass** es sich um eine organische Elektrolumineszenzvorrichtung handelt und die Verbindung nach einem oder mehreren der Ansprüche 1 bis 12 als Matrixmaterial für eine phosphoreszierende Verbindung in einer emittierenden Schicht eingesetzt wird.

## Claims

1. Compound of the formula (1), 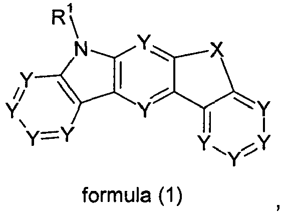 where the following applies to the symbols used:
Y is on each occurrence, identically or differently, CR or N;
X is selected from C(R¹)₂, O, S, PR¹, P(=O)R¹ or BR¹;
R, R¹ are on each occurrence, identically or differently, H, D, F, Cl, Br, I, N(R²)₂, N(Ar)₂, C(=O)Ar, P(=O)Ar₂, S(=O)Ar, S(=O)₂Ar, CR²=CR²Ar, CN, NO₂, Si(R²)₃, B(OR²)₂, OSO₂R², a straight-chain alkyl, alkoxy or thioalkoxy group having 1 to 40 C atoms or a branched or cyclic alkyl, alkoxy or thioalkoxy group having 3 to 40 C atoms, each of which may be substituted by one or more radicals R², where one or more non-adjacent CH₂ groups may be replaced by R²C=CR², C≡C, Si(R²)₂, C=O, C=NR², P(=O)(R²), SO, SO₂, NR², O, S or CONR² and where one or more H atoms may be replaced by D, F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 40 aromatic ring atoms, which may be substituted by one or more radicals R², or an aralkyl or heteroaralkyl group having 5 to 40 aromatic ring atoms, which may be substituted by one or more radicals R², or a combination of these systems; two or more substituents R here, together with the atoms to which they are bonded, or two substituents R¹, together with the atom to which they are bonded, may also form a mono- or polycyclic, aliphatic or aromatic ring system with one another;
Ar is on each occurrence, identically or differently, an aromatic or heteroaromatic ring system having 5 to 40 aromatic ring atoms, which may be substituted by one or more radicals R³;
R² is on each occurrence, identically or differently, H, D, F, Cl, Br, I, N(R³)₂, N(Ar)₂, C(=O)Ar, P(=O)Ar₂, S(=O)Ar, S(=O)₂Ar, CR³=CR³Ar, CN, NO₂, Si(R³)₃, B(OR³)₂, OSO₂R³, a straight-chain alkyl, alkoxy or thioalkoxy group having 1 to 40 C atoms or a branched or cyclic alkyl, alkoxy or thioalkoxy group having 3 to 40 C atoms, each of which may be substituted by one or more radicals R³, where one or more non-adjacent CH₂ groups may be replaced by R³C=CR³, C≡C, Si(R³)₂, C=O, C=NR³, P(=O)(R³), SO, SO₂, NR³, O, S or CONR³ and where one or more H atoms may be replaced by D, F, Cl, Br, I, CN or NO₂, or an aromatic or heteroaromatic ring system having 5 to 60 aromatic ring atoms, which may in each case be substituted by one or more radicals R³, or an aryloxy or heteroaryloxy group having 5 to 40 aromatic ring atoms, which may be substituted by one or more radicals R³, or an aralkyl or heteroaralkyl group having 5 to 40 aromatic ring atoms, which may be substituted by one or more radicals R³, or a combination of these systems;
R³ is on each occurrence, identically or differently, H, D or an aliphatic hydrocarbon radical having 1 to 20 C atoms or an aryl or heteroaryl group having 5 to 40 ring atoms or a combination of these groups;
with the proviso that, if one or more of the groups R, R¹, R², R³, Ar or Ar¹ contain heteroaryl groups which do not conform to the formulae (2) or (3), these are not electron-deficient heteroaryl groups, where an electron-deficient heteroaryl group is a 5-membered heteroaryl ring group having at least two heteroatoms or 6-membered heteroaryl ring group having at least one heteroatom and further 6-membered aryl ring groups or 6-membered heteroaryl ring groups may be condensed onto these groups;
**characterised in that** at least one group R is present which stands, identically or differently on each occurrence, for a group of the following formula (2), 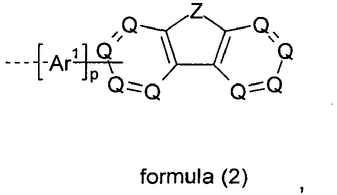 where the dashed bond indicates the linking of the group of the formula (2), R² has the above-mentioned meanings, and furthermore:
Q is C if the group of the formula (2) is linked to Ar¹ or to the remainder of the molecule via this group; or is, identically or differently on each occurrence, CR² or N in the other cases;
Z is NR²;
Ar¹ is a divalent aromatic or heteroaromatic ring system having 5 to 40 aromatic ring atoms, which may be substituted by one or more radicals R²;
p is 0 or 1;
and/or **in that** at least one group R¹ is present which stands for a group of the following formula (3), 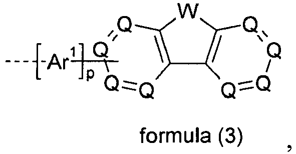 where the dashed bond indicates the linking of the group of the formula (3), R², Ar¹, Q and p have the above-mentioned meanings, and furthermore:
W is NR², O or S;
compounds of the following general formula are excluded from the invention: 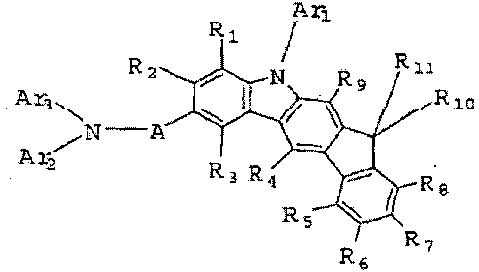 where the following applies to the symbols used:
A is a divalent substituted or unsubstituted aromatic hydrocarbon group, a divalent substituted or unsubstituted aromatic heterocyclic ring or a divalent substituted or unsubstituted condensed polycyclic aromatic group;
Ar₁, Ar₂, Ar₃ are identical or different and stand for a substituted or unsubstituted aromatic hydrocarbon group, a substituted or unsubstituted aromatic heterocyclic group or a substituted or unsubstituted condensed polycyclic aromatic group, where A und Ar₂ are bonded to one another via a single bond and thus form a ring:
R₁ bis R₉ are identical or different and stand for a hydrogen atom, a deuterium atom, a fluorine atom, a chlorine atom, cyano, nitro, linear or branched alkyl having 1 to 6 carbon atoms, which may carry a substituent, cycloalkyl having 5 to 10 carbon atoms, which may carry a substituent, linear or branched alkenyl having 2 to 6 carbon atoms, which may carry a substituent, linear or branched alkyloxy having 1 to 6 carbon atoms, which may carry a substituent, cycloalkyloxy having 5 to 10 carbon atoms, which may carry a substituent, a substituted or unsubstituted aromatic hydrocarbon group, a substituted or unsubstituted aromatic heterocyclic group, a substituted or unsubstituted condensed polycyclic aromatic group or substituted or unsubstituted aryloxy, which may be bonded to one another via a single bond, substituted or unsubstituted methylene, an oxygen atom or a sulfur atom and thus form a ring; and
R₁₀ und R₁₁ are identical or different and stand for linear or branched alkyl having 1 to 6 carbon atoms, which may carry a substituent, cycloalkyl having 5 to 10 carbon atoms, which may carry a substituent, linear or branched alkenyl having 2 to 6 carbon atoms, which may carry a substituent, linear or branched alkyloxy having 1 to 6 carbon atoms, which may carry a substituent, cycloalkyloxy having 5 to 10 carbon atoms, which may carry a substituent, a substituted or unsubstituted aromatic hydrocarbon group, a substituted or unsubstituted aromatic heterocyclic group, a substituted or unsubstituted condensed polycyclic aromatic group or substituted or unsubstituted aryloxy, which may be bonded to one another via a single bond, substituted or unsubstituted methylene, an oxygen atom or a sulfur atom and thus form a ring.

2. Compound according to Claim 1, **characterised in that** the radical R¹ which is bonded to the nitrogen atom stands for an aromatic or heteroaromatic ring system having 5 to 60 aromatic ring atoms, which may also be substituted by one or more radicals R², or for a group of the formula (3).

3. Compound according to Claim 1 or 2, **characterised in that** X stands for C(R¹)₂, where R¹ stands, identically or differently on each occurrence, for a straight-chain alkyl group having 1 to 10 C atoms or a branched or cyclic alkyl group having 3 to 10 C atoms, each of which may be substituted by one or more radicals R², where one or more non-adjacent CH₂ groups may be replaced by R²C=CR², C≡C, Si(R²)₂, C=O, O, S or CONR² and where one or more H atoms may be replaced by D, F or CN, or for an aromatic or heteroaromatic ring system having 5 to 60 aromatic ring atoms, which may also be substituted by one or more radicals R²; the radicals R¹ here may also form an aromatic or aliphatic ring system with one another.

4. Compound according to one or more of Claims 1 to 3, **characterised in that** a maximum of one group Y per ring stands for N and the remaining groups Y stand, identically or differently on each occurrence, for CR.

5. Compound according to one or more of Claims 1 to 4, selected from compounds of the formula (5), 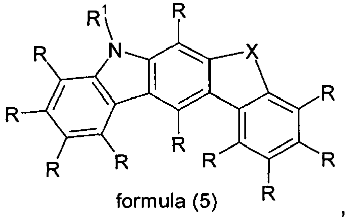 where the symbols used have the meanings given in Claim 1.

6. Compound according to Claim 5, selected from the structures of the formulae (6), (7) and (8), 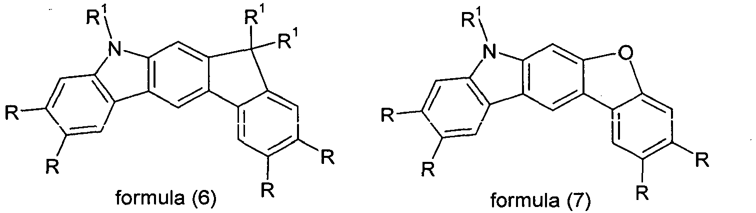 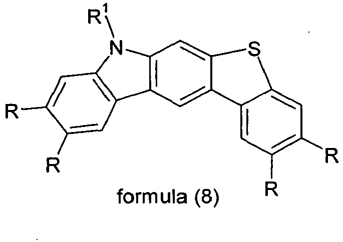 where the symbols used have the meanings given in Claim 1.

7. Compound according to Claim 6, selected from the compounds of the formulae (6b), (7b) and (8b), 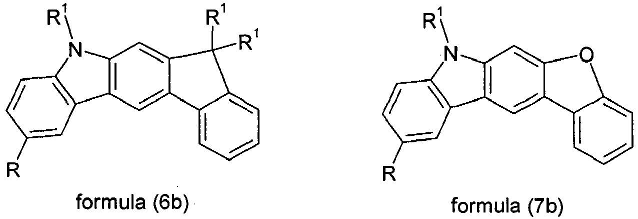 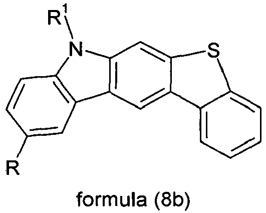 where the symbols used have the meanings given in Claim 1.

8. Compound according to one or more of Claims 1 to 7, **characterised in that** the groups of the formulae (2) to (3) are selected from the groups of the formulae (2a) to (3a), 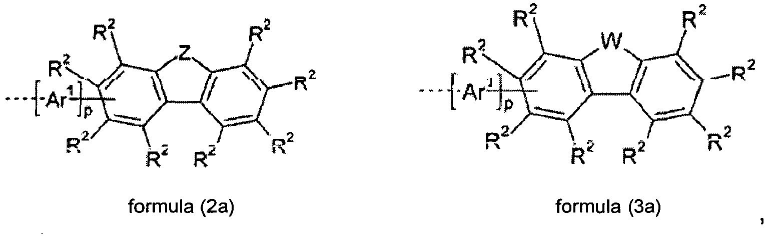 where the symbols and indices used have the meanings given in Claim 1, and, in formulae (2a) and (3a), no group R² is bonded at the position at which the group is linked to Ar¹ or the remainder of the molecule.

9. Compound according to one or more of Claims 1 to 8, **characterised in that** the groups of the formulae (2) to (4) are selected from the structures of the formulae (2b), (2c), (2d), (3b), (3c) and (3d), 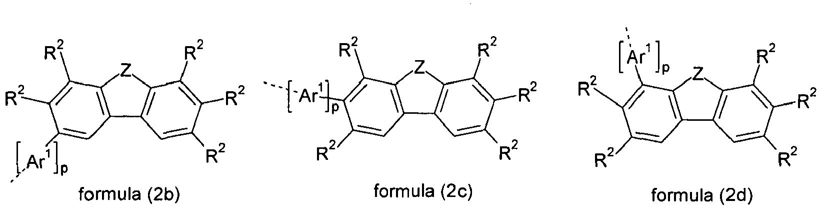 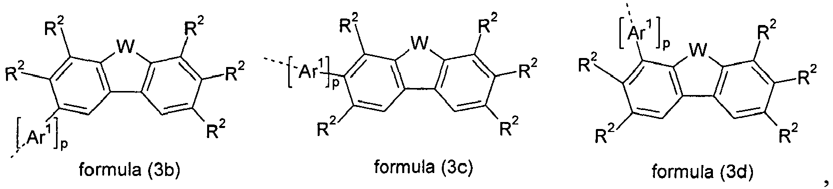 where the dashed bond indicates the linking of the group to the remainder of the molecule and the other symbols and indices used have the meanings given in Claim 1.

10. Compound according to Claim 8 or 9, **characterised in that** the radicals R² which are bonded to a carbon atom in formulae (2a) to (3d) stand for H.

11. Compound according to one or more of Claims 1 to 10, **characterised in that** Z or W stands for NR², where R² stands for an aromatic or heteroaromatic ring system.

12. Compound according to one or more of Claims 1 to 11, selected from structures of the formula (9), (10), (11) or (12), 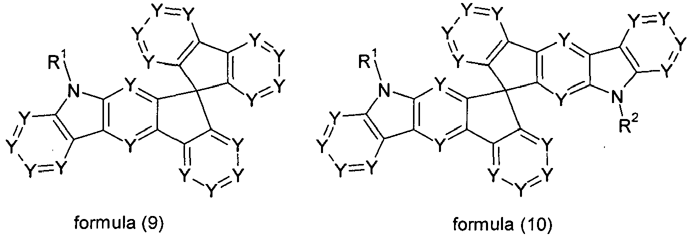 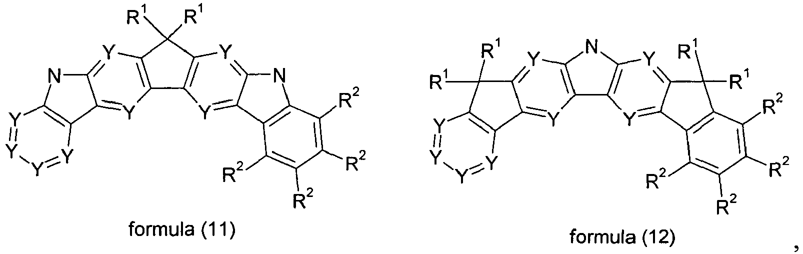 where the symbols used have the meanings given in Claim 1.

13. Process for the preparation of a compound according to one or more of Claims 1 to 12, **characterised in that** the group of the formula (2) or (3) is introduced by a Suzuki coupling, an Ullmann coupling or by a Hartwig-Buchwald coupling.

14. Mixture comprising at least one compound according to one or more of Claims 1 to 12 and at least one fluorescent or phosphorescent dopant.

15. Formulation, in particular a solution, a suspension or a miniemulsion, comprising at least one compound according to one or more of Claims 1 to 12 or a mixture according to Claim 14 and one or more solvents.

16. Use of a compound according to one or more of Claims 1 to 12 or a mixture according to Claim 14 in an electronic device.

17. Electronic device, in particular selected from the group consisting of organic electroluminescent devices, organic integrated circuits, organic field-effect transistors, organic thin-film transistors, organic light-emitting transistors, organic solar cells, organic dye-sensitised solar cells, organic optical detectors, organic photoreceptors, organic field-quench devices, light-emitting electrochemical cells, organic laser diodes and "organic plasmon emitting devices", containing at least one compound according to one or more of Claims 1 to 12 or a mixture according to Claim 14.

18. Electronic device according to Claim 17, **characterised in that** it is an organic electroluminescent device and the compound according to one or more of Claims 1 to 12 is employed as matrix material for a phosphorescent compound in an emitting layer.

## Revendications

1. Composé de la formule (1) : 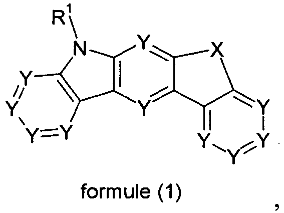 dans laquelle ce qui suit s'applique aux symboles utilisés :
Y est pour chaque occurrence, de manière identique ou différente, CR ou N ;
X est choisi parmi C(R¹)₂, O, S, PR¹, P(=O)R¹ ou BR¹ ;
R, R¹ sont pour chaque occurrence, de manière identique ou différente, H, D, F, Cl, Br, I, N(R²)₂, N(Ar)₂, C(=O)Ar, P(=O)Ar₂, S(=O)Ar, S(=O)₂Ar, CR²=CR²Ar, CN, NO₂, Si(R²)₃, B(OR²)₂, OSO₂R², un groupe alkyle, alcoxy ou thioalcoxy en chaîne droite comportant 1 à 40 atome(s) de C ou un groupe alkyle, alcoxy ou thioaicoxy ramifié ou cyclique comportant 3 à 40 atomes de C, dont chacun peut être substitué par un radical ou plusieurs radicaux R², où un ou plusieurs groupe(s) CH₂ non adjacents peut/ peuvent être remplacé(s) par R²C=CR², C=C, Si(R²)₂, C=O, C=NR², P(=O)(R²), SO, SO₂, NR², O, S ou CONR² et où un ou plusieurs atome(s) de H peut/peuvent être remplacé(s) par D, F, Cl, Br, I, CN ou NO₂, ou un système de cycle aromatique ou hétéroaromatique qui comporte 5 à 60 atomes de cycle aromatique, lequel peut dans chaque cas être substitué par un radical ou plusieurs radicaux R², ou un groupe aryloxy ou hétéroaryloxy qui comporte 5 à 40 atomes de cycle aromatique, lequel peut être substitué par un radical ou plusieurs radicaux R², ou un groupe aralkyle ou hétéroaralkyle qui comporte 5 à 40 atomes de cycle aromatique, lequel peut être substitué par un radical ou plusieurs radicaux R², ou une combinaison de ces systèmes; deux substituants R ou plus ici, en association avec les atomes auxquels ils sont liés, ou deux substituants R¹, en association avec l'atome auquel ils sont liés, peuvent également former un système de cycle aliphatique ou aromatique monocyclique ou polycyclique l'un avec l'autre ou les uns avec les autres ;
Ar est pour chaque occurrence, de manière identique ou différente, un système de cycle aromatique ou hétéroaromatique qui comporte 5 à 40 atomes de cycle aromatique, lequel peut être substitué par un radical ou plusieurs radicaux R³ ;
R² est pour chaque occurrence, de manière identique ou différente, H, D, F, Cl, Br, I, N(R³)₂, N(Ar)₂, C(=O)Ar, P(=O)Ar₂, S(=O)Ar, S(=O)₂Ar, CR³=CR³Ar, CN, NO₂, Si(R³)₃, B(OR³)₂, OSO₂R³, un groupe alkyle, alcoxy ou thioalcoxy en chaîne droite qui comporte 1 à 40 atome(s) de C ou un groupe alkyle, alcoxy ou thioalcoxy ramifié ou cyclique qui comporte 3 à 40 atomes de C, dont chacun peut être substitué par un radical ou plusieurs radicaux R³, où un ou plusieurs groupe(s) CH₂ non adjacents peut/ peuvent être remplacé(s) par R³C=CR³, C≡C, Si(R³)₂, C=O, C=NR³, P(=O)(R³), SO, SO₂, NR³, O, S ou CONR³ et où un ou plusieurs atome(s) de H peut/peuvent être remplacé(s) par D, F, Cl, Br, I, CN ou NO₂, ou un système de cycle aromatique ou hétéroaromatique qui comporte 5 à 60 atomes de cycle aromatique, lequel peut dans chaque cas être substitué par un radical ou plusieurs radicaux R³, ou un groupe aryloxy ou hétéroaryloxy qui comporte 5 à 40 atomes de cycle aromatique, lequel peut être substitué par un radical ou plusieurs radicaux R³, ou un groupe aralkyle ou hétéroaralkyle qui comporte 5 à 40 atomes de cycle aromatique, lequel peut être substitué par un radical ou plusieurs radicaux R³, ou une combinaison de ces systèmes ;
R³ est pour chaque occurrence, de manière identique ou différente, H, D ou un radical hydrocarbone aliphatique qui comporte 1 à 20 atome(s) de C ou un groupe aryle ou hétéroaryle qui comporte 5 à 40 atomes de cycle ou une combinaison de ces groupes ;
étant entendu que, si un ou plusieurs des groupes R, R¹, R², R³, Ar ou Ar¹ contient/contiennent des groupes hétéroaryle qui ne sont pas conformes aux formules (2) ou (3), ce ne sont pas des groupes hétéroaryle déficients en électrons, où un groupe hétéroaryle déficient en électrons est un groupe de cycle hétéroaryle à 5 éléments qui comporte au moins deux hétéroatomes ou un groupe de cycle hétéroaryle à 6 éléments qui comporte au moins un hétéroatome et en outre, des groupes de cycle aryle à 6 éléments ou des groupes de cycle hétéroaryle à 6 éléments peuvent être condensés sur ces groupes ;
**caractérisé en ce qu'**au moins un groupe R est présent, lequel représente, de manière identique ou différente pour chaque occurrence, un groupe de la formule (2) qui suit : 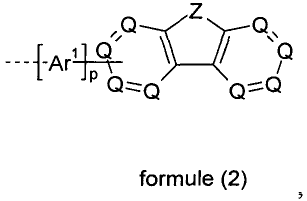 dans laquelle le lien en pointillés indique la liaison du groupe de la formule (2), R² présente les significations mentionnées ci avant, et en outre :
Q est C si le groupe de la formule (2) est lié à Ar¹ ou au reste de la molécule via ce groupe ; ou est, de manière identique ou différente pour chaque occurrence, CR² ou N dans les autres cas ;
Z est NR² ;
Ar¹ est un système de cycle aromatique ou hétéroaromatique divalent qui comporte 5 à 40 atomes de cycle aromatique, lequel peut être substitué par un radical ou plusieurs radicaux R² ;
p est 0 ou 1 ;
et/ou **en ce qu'**au moins un groupe R¹ est présent, lequel représente un groupe de la formule (3) qui suit : 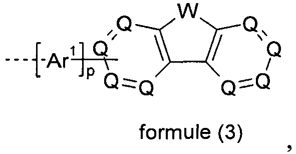 où le lien en pointillés indique la liaison du groupe de la formule (3), R², Ar¹, Q et p présentent les significations mentionnées ci avant, et en outre :
W est NR², O ou S ;
les composés de la formule générale qui suit sont exclus de l'invention : 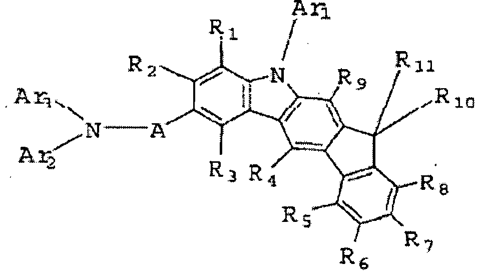 dans laquelle ce qui suit s'applique aux symboles utilisés :
A est un groupe hydrocarbone aromatique substitué ou non substitué divalent, un groupe hétérocyclique aromatique substitué ou non substitué divalent ou un groupe aromatique polycyclique condensé substitué ou non substitué divalent ;
Ar₁, Ar₂, Ar₃ sont identiques ou différents et représentent un groupe hydrocarbone aromatique substitué ou non substitué, un groupe hétérocyclique aromatique substitué ou non substitué ou un groupe aromatique polycyclique condensé substitué ou non substitué, où A et Ar₂ sont liés l'un à l'autre via une liaison simple et ils forment par conséquent un cycle ;
R₁ à R₉ sont identique ou différents et représentent un atome d'hydrogène, un atome de deutérium, un atome de fluor, un atome de chlore, un radical cyano, un radical nitro, alkyle linéaire ou ramifié qui comporte 1 à 6 atome(s) de carbone, lequel peut porter un substituant, cycloalkyle qui comporte 5 à 10 atomes de carbone, lequel peut porter un substituant, alkényle linéaire ou ramifié qui comporte 2 à 6 atomes de carbone, lequel peut porter un substituant, alkyloxy linéaire ou ramifié qui comporte 1 à 6 atome(s) de carbone, lequel peut porter un substituant, cycloalkyloxy qui comporte 5 à 10 atomes de carbone, lequel peut porter un substituant, un groupe hydrocarbone aromatique substitué ou non substitué, un groupe hétérocyclique aromatique substitué ou non substitué, un groupe aromatique polycyclique condensé substitué ou non substitué ou aryloxy substitué ou non substitué, lesquels peuvent être liés les uns aux autres via une liaison simple, méthylène substitué ou non substitué, un atome d'oxygène ou un atome de soufre et peuvent ainsi former un cycle ; et
R₁₀ et R₁₁ sont identiques ou différents et représentent alkyle linéaire ou ramifié qui comporte 1 à 6 atome(s) de carbone, lequel peut porter un substituant, cycloalkyle qui comporte 5 à 10 atomes de carbone, lequel peut porter un substituant, alkényle linéaire ou ramifié qui comporte 2 à 6 atomes de carbone, lequel peut porter un substituant, alkyloxy linéaire ou ramifié qui comporte 1 à 6 atome(s) de carbone, lequel peut porter un substituant, cycloalkyloxy qui comporte 5 à 10 atomes de carbone, lequel peut porter un substituant, un groupe hydrocarbone aromatique substitué ou non substitué, un groupe hétérocyclique aromatique substitué ou non substitué, un groupe aromatique polycyclique condensé substitué ou non substitué ou aryloxy substitué ou non substitué, lesquels peuvent être liés les uns aux autres via une liaison simple, méthylène substitué ou non substitué, un atome d'oxygène ou un atome de soufre et peuvent ainsi former un cycle.

2. Composé selon la revendication 1, **caractérisé en ce que** le radical R¹ qui est lié à l'atome d'azote représente un système de cycle aromatique ou hétéroaromatique qui comporte 5 à 60 atomes de cycle aromatique, lequel peut également être substitué par un radical ou plusieurs radicaux R², ou un groupe de la formule (3).

3. Composé selon la revendication 1 ou 2, **caractérisé en ce que** X représente C(R¹)₂, où R¹ représente, de manière identique ou différente pour chaque occurrence, un groupe alkyle en chaîne droite qui comporte 1 à 10 atome(s) de C ou un groupe alkyle ramifié ou cyclique qui comporte 3 à 10 atomes de C, dont chacun peut être substitué par un radical ou plusieurs radicaux R², où un ou plusieurs groupe(s) CH₂ non adjacents peut/peuvent être remplacé(s) par R²C=CR², C≡C, Si(R²)₂, C=O, O, S ou CONR² et où un ou plusieurs atome(s) de H peut/ peuvent être remplacé(s) par D, F or CN, ou un système de cycle aromatique ou hétéroaromatique qui comporte 5 à 60 atomes de cycle aromatique, lequel peut également être substitué par un radical ou plusieurs radicaux R²; les radicaux R¹ ici peuvent également former un système de cycle aromatique ou aliphatique les uns avec les autres.

4. Composé selon une ou plusieurs des revendications 1 à 3, **caractérisé en ce qu'**un maximum d'un groupe Y par cycle représente N et les groupes Y restants représentent, de manière identique ou différente pour chaque occurrence, CR.

5. Composé selon une ou plusieurs des revendications 1 à 4, choisi parmi les composés de la formule (5) : 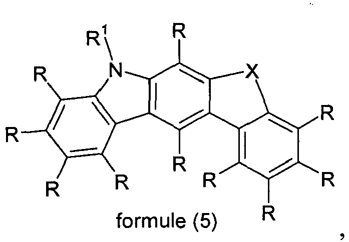 dans laquelle les symboles utilisés présentent les significations données selon la revendication 1.

6. Composé selon la revendication 5, choisi parmi les structures des formules (6), (7) et (8) : 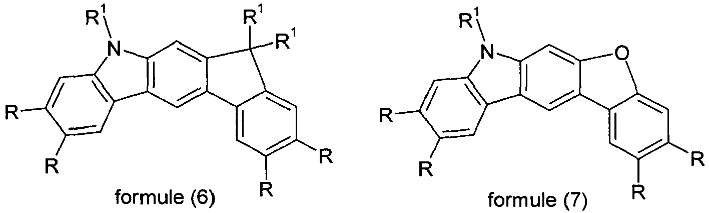 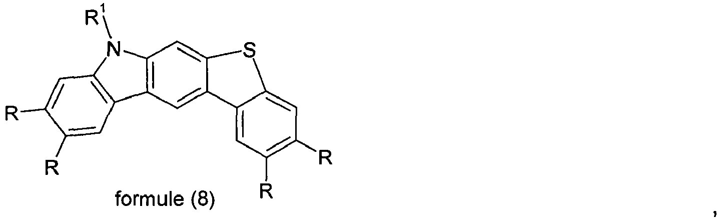 dans lesquelles les symboles utilisés présentent les significations données selon la revendication 1.

7. Composé selon la revendication 6, choisi parmi les composés des formules (6b), (7b) et (8b) : 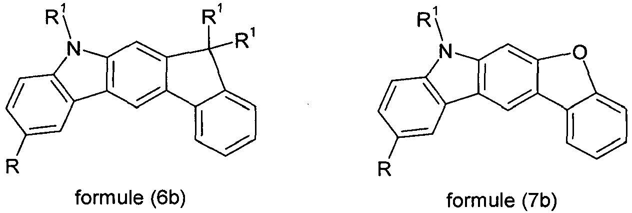 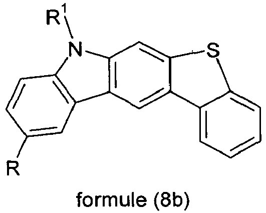 dans lesquelles les symboles utilisés présentent les significations données selon la revendication 1.

8. Composé selon une ou plusieurs des revendications 1 à 7, **caractérisé en ce que** les groupes des formules (2) à (3) sont choisis parmi les groupes des formules (2a) et (3a) : 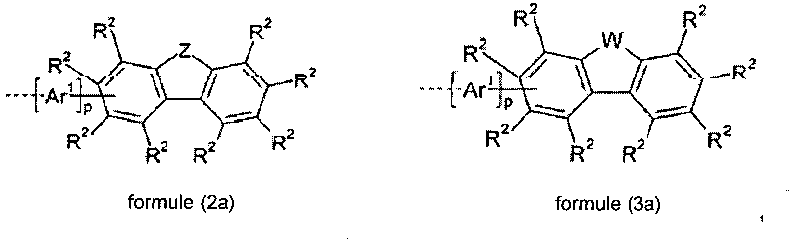 dans lesquelles les symboles et indices utilisés présentent les significations données selon la revendication 1, et, dans les formules (2a) et (3a), aucun groupe R² n'est lié à la position au niveau de laquelle le groupe est lié à Ar¹ ou au reste de la molécule.

9. Composé selon une ou plusieurs des revendications 1 à 8, **caractérisé en ce que** les groupes des formules (2) à (4) sont choisis parmi les structures des formules (2b), (2c), (2d), (3b), (3c) et (3d) : 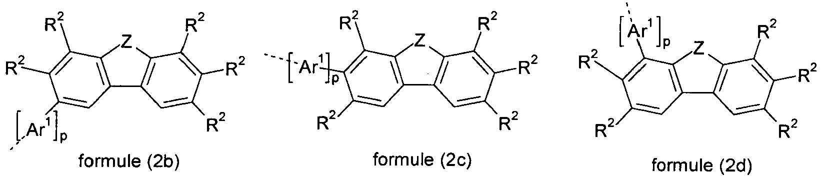 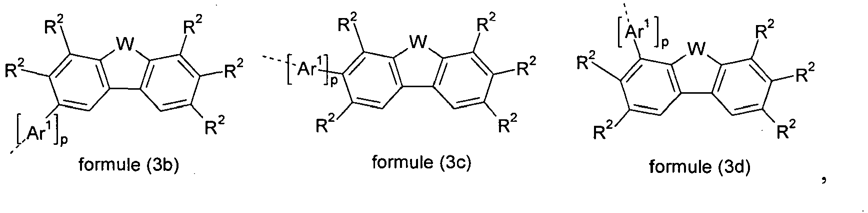 dans lesquelles le lien en pointillés indique la liaison du groupe sur le reste de la molécule et les autres symboles et indices utilisés présentent les significations données selon la revendication 1.

10. Composé selon la revendication 8 ou 9, **caractérisé en ce que** les radicaux R² qui sont liés à un atome de carbone dans les formules (2a) à (3d) représentent H.

11. Composé selon une ou plusieurs des revendications 1 à 10, **caractérisé en ce que** Z ou W représente NR², où R² représente un système de cycle aromatique ou hétéroaromatique.

12. Composé selon une ou plusieurs des revendications 1 à 11, choisi parmi les structures des formules (9), (10), (11) ou (12) : 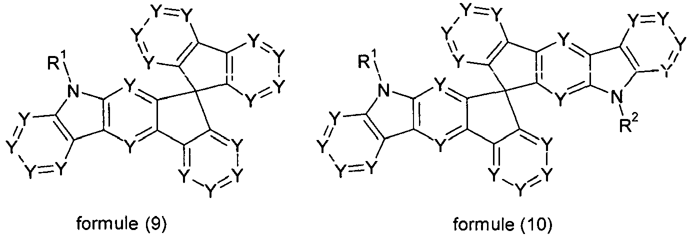 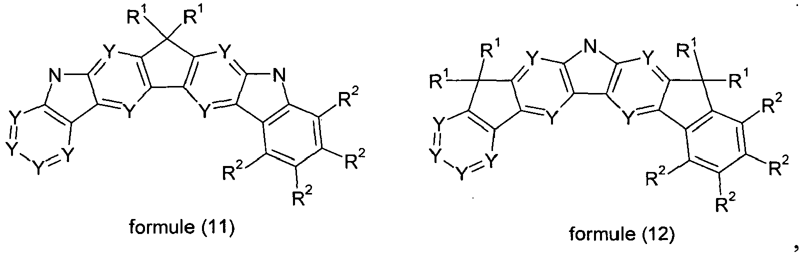 dans lesquelles les symboles utilisés présentent les significations données selon la revendication 1.

13. Procédé pour la préparation d'un composé selon une ou plusieurs des revendications 1 à 12, **caractérisé en ce que** le groupe de la formule (2) ou (3) est introduit au moyen d'un couplage de Suzuki, au moyen d'un couplage d'Ullmann ou au moyen d'un couplage de Hartwig-Buchwald.

14. Mélange comprenant au moins un composé selon une ou plusieurs des revendications 1 à 12 et au moins un dopant fluorescent ou phosphorescent.

15. Formulation, en particulier une solution, une suspension ou une mini-émulsion, comprenant au moins un composé selon une ou plusieurs des revendications 1 à 12 ou un mélange selon la revendication 14 et un ou plusieurs solvant(s).

16. Utilisation d'un composé selon une ou plusieurs des revendications 1 à 12 ou d'un mélange selon la revendication 14 dans un dispositif électronique.

17. Dispositif électronique, en particulier choisi parmi le groupe constitué par les dispositifs électroluminescents organiques, les circuits intégrés organiques, les transistors à effet de champ organiques, les transistors à film mince organiques, les transistors à émission de lumière organiques, les cellules solaires organiques, les cellules solaires sensibilisées par colorant organiques, les détecteurs optiques organiques, les photorécepteurs organiques, les dispositifs à extinction de champ organiques, les cellules électrochimiques à émission de lumière, les diodes laser organiques et les "dispositifs à émission de plasmons organiques", contenant au moins un composé selon une ou plusieurs des revendications 1 à 12 ou un mélange selon la revendication 14.

18. Dispositif électronique selon la revendication 17, **caractérisé en ce qu'**il s'agit d'un dispositif électroluminescent organique et le composé selon une ou plusieurs des revendications 1 à 12 est utilisé en tant que matériau de matrice pour un composé phosphorescent dans une couche d'émission.
